# EUROPEAN PATENT SPECIFICATION

(11) **EP 4 385 389 B1**
(45) Date of publication and mention of the grant of the patent: **25.03.2026**
(21) Application number: 22383232.0
(22) Date of filing: 16.12.2022
(51) Int. Cl.: A61B 1/00, A61B 1/015, A61M 1/00

(54) **MEDICAL DEVICE AND MEDICAL KIT, IN PARTICULAR FOR CARRYING OUT AN INTRACORPORAL VACUUM THERAPY**
MEDIZINISCHE VORRICHTUNG UND MEDIZINISCHES SET, INSBESONDERE ZUR DURCHFÜHRUNG EINER INTRAKORPORALEN VAKUUMTHERAPIE
DISPOSITIF MÉDICAL ET KIT MÉDICAL, EN PARTICULIER POUR LA MISE EN OEUVRE D'UNE THÉRAPIE INTRACORPORELLE SOUS VIDE

(43) Date of publication of application: 19.06.2024
(73) Proprietor: Boston Scientific Medical Device Limited, CO Galway (IE)
(72) Inventor: ARDEVOL, Jordi, 08191 Rubí (Barcelona) (ES); TURÓN DOLS, Pau, 08191 Rubí (Barcelona) (ES); GRANDES VILACLARA, Maria Eugenia, 08191 Rubí (Barcelona) (ES); SANCHEZ GARRIDO, Ricardo, 08191 Rubí (Barcelona) (ES); HARARI, Shahar, 6941154 Tel Aviv (IL); SHENHAV, Avshalom, 3220207 Haifa (IL); GOLDENBERG, Nir, 3435331 Haifa (IL); TROMER, Dotan, 2518000 Moshav Hosen (IL); HADAS, Arnon, 83800 Moshav Avigdor (IL); GREENBERG, Kobby, 4050000 Even Yehuda (IL)
(74) Representative: Vossius & Partner Patentanwälte Rechtsanwälte mbB

(56) References cited:
- EP-A2- 2 512 318
- WO-A1-2011/038949
- WO-A1-2012/123414
- WO-A1-2018/029229

## Description

### FIELD OF INVENTION AND TECHNICAL BACKGROUND

The present invention relates to a medical kit comprising a medical device, in particular for carrying out an intracorporal, preferably endoluminal and/or endocavitary, vacuum therapy.

Intracorporal vacuum therapy requires reaching the cavity to be treated, for example in the esophagus or colon, with an endoscope and the deployment of a drainage device in a precise location to improve the performance of the vacuum therapy. Nowadays, with the aid of an endoscope, an overtube is placed inside the cavity. Afterwards, the endoscope is removed, while the overtube is left inside the patient's body, thereby providing a pathway from the outside to the internal area to be treated. To implement the vacuum therapy, a fluid drainage device containing a sponge is pushed through the overtube and placed inside the patient's cavity, promoting debridement of the cavity and formation of granulation tissue. The drainage device is configured for draining body fluids accumulated inside the cavity. For this purpose, the fluid drainage device is typically connected to a negative pressure source being outside the patient's body. An example of this type of vacuum therapy is known from EP 1 572 286 B1. An example of a prior art document disclosing an endoscope sheath to aid in the delivery of devices to a selected target area in the anatomy of a patient is EP 2 512 318 B1.

However, the vacuum therapy procedures described in the prior art face several limitations. Firstly, the fluid drainage device must be implanted following a visionless deployment technique, as the presence of the endoscope is not compatible with the introduction of the drainage device. Consequently, the accurate placement of the fluid drainage device needs to be checked in a second introduction of the endoscope. Secondly, the semi-rigid nature of the overtube restricts the movement of the distal part of the endoscope. As a result, angulated cavities with difficult access cannot be reached, limiting the treatment of such cavities. In addition, the overtube might be dislocated while pushing the fluid drainage device through it. This situation poses a risk of damaging adjacent tissues or enlarging the orifice to access the cavity. Another shortcoming of current delivery systems relates to the high friction generated between the sponge of the fluid drainage device and the inner side of the overtube which leads to the use of large quantities of lubricant.

In view of the above, addressing the limitations of current methods represents an unmet need in the field of intracorporeal vacuum therapies.

### OBJECT AND SOLUTION

The object of the present invention is to provide a medical kit which at least partially address the problems of the prior art, in particular facilitates the execution of intracorporeal vacuum therapies by allowing a more precise and gentler positioning of a fluid drainage device, thereby minimizing the risk for patients undergoing these procedures.

This object is properly addressed by a medical kit as defined in independent claim 1. Preferred embodiments are defined in the dependent claims.

Further disclosed but not forming part for the invention as claimed are a sheath and a method for carrying out a vacuum therapy as defined in the description.

According to a first aspect, the present disclosure refers to a medical device, in particular for carrying out an intracorporal, preferably endoluminal and/or endocavitary, vacuum therapy, preferably for draining body fluids, preferably from intracorporal cavities such as esophagus, colon, stomach or fistula (such as anal fistula).

The medical device comprises
- a first flexible, in particular compressible and/or pliant, tube having a proximal end portion and a distal end portion
   and
- a second flexible, in particular compressible and/or pliant, tube having a proximal end portion and a distal end portion.

The first flexible tube is adapted to insert an endoscope into a patient's body.

The second flexible tube is adapted to insert a fluid drainage device, in particular a fluid drainage device being at least partially or sectionally, in particular only partially or sectionally or completely, surrounded by a sheath, in particular in the form of a capsule, into a patient's body, wherein the fluid drainage device is adapted to drain body fluids.

Further, the first flexible tube and the second flexible tube are, in particular at least partially or sectionally, in particular only partially or sectionally or continuously, connected or joined together lengthwise, i.e. lengthways or longitudinal.

The term "body fluids" as used according to the present invention refers to body fluids such as blood, lymphe, ichors, exudates, purulent fluids, or intestinal contents. Particularly, the term "body fluids" as used according to the present invention may refer to body fluids which are typically present in an abscess and/or fistula.

The term "fistula" as used according to the present invention refers to an abnormal anastomosis, that is, an abnormal connection between two hollow spaces (technically, two epithelialized surfaces), such as blood vessel, intestines or other hollow organs, for example esophagus or colon.

The term "proximal end portion" as used according to the present invention refers to an end portion facing toward the trunk of a user, in particular physician or medical staff.

The term "distal end portion" as used according to the present invention refers to an end portion facing away from the trunk of a user, in particular physician or medical staff.

The term "patient's body" as used according to the present invention preferably refers to the body of a human or other mammal, i.e. to the body of a human mammal or non-human mammal.

The term "intracorporal" as used according to the present invention preferably refers to an anatomical area located inside the patient's body, in particular being accessible by an endoscope.

Preferably, the term "endoscope" as used according to the present invention refers to a flexible, in particular compressible and/or pliant, endoscope, in particular hand-held endoscope.

More specifically, the first flexible tube and the second flexible tube may be connected together lengthwise with a material bond, i.e. in a materially bonded manner. For example, the first flexible tube and the second flexible tube may be glued, welded or sewed together lengthwise.

Preferably, the first flexible tube and the second flexible tube are, in particular at least partially or sectionally, preferably only partially or sectionally or continuously, arranged parallel to each other.

Further, the first flexible tube may have a length of 75 mm to 800 mm, in particular 100 mm to 700 mm, preferably 150 mm to 600 mm. For example, if the medical device is provided for a treatment of the esophagus, the medical device may have a length of 490 mm. Alternatively, for example, if the medical device is provided for a treatment of another intracorporal site, e.g. a lower gastrointestinal tract, the medical device may have a length of 200 mm.

Further, the first flexible tube may have a cornerless, i.e. round, in particular circular, oval or elliptic, cross-section. Alternatively, the first flexible tube may have a polygonal, in particular triangular, rectangular, square, trapezoid, rhomboid, pentagonal, hexagonal, heptagonal, octagonal, nonagonal, decagonal or star-like, cross-section.

Further, the first flexible tube may have an inner diameter of 8 mm to 40 mm, in particular 13 mm to 30 mm, preferably 16 mm to 28 mm. For example, the first flexible tube may have an inner diameter of 20 mm.

Further, the first flexible tube may have a wall thickness, in particular film wall thickness, of 50 µm to 500 µm, in particular 60 µm to 350 µm, preferably 70 µm to 250 µm. For example, the first flexible tube may have a wall thickness of 80 µm.

Further, the first flexible tube may comprise or consist of a polymer, in particular a natural polymer and/or synthetic polymer and/or in vivo degradable or resorbable polymer, in particular selected from group consisting of polyolefin, polyethylene, polypropylene, polyvinyl chloride, polytetrafluorethylene, polyurethane, polyether block amide, polycarbonate, ethylene vinyl acetate, polyester, polyacrylate, polyether ether ketone, polyethylene terephthalate, polyethylene terephthalate glycol, silicone, chitosan, cellulose, silk, gellan gum, polycaprolactone, polylactic acid, polyglycolide, polylactic-co-glycolic acid, polyvinyl alcohol, polyhydroxyalkanoate, poly-3-hydroxybutyrate, polytrimethylene carbonate, copolymers of at least two of the afore-said polymers and mixtures of at least two of the afore-said polymers.

The term "copolymer" as used according to the present invention refers to a polymer comprising or consisting of at least two different monomer units, in particular at least two different repeating monomer units. Accordingly, the term "copolymer" as used according to the present invention may encompass, for example, bipolymers, terpolymers, tetrapolymers, and the like.

Further, the second flexible tube may have a length of 130 mm to 1000 mm, in particular 200 mm to 850 mm, preferably 250 mm to 700 mm. For example, if the medical device is provided for a treatment of the esophagus, the medical device may have a length of 550 mm. Alternatively, for example, if the medical device is provided for a treatment of another intracorporal site, e.g. a lower gastrointestinal tract, the medical device may have a length of 300 mm.

Further, the second flexible tube may have a cornerless, i.e. round, in particular circular, oval or elliptic, cross-section. Alternatively, the second flexible tube may have a polygonal, in particular triangular, rectangular, square, trapezoid, rhomboid, pentagonal, hexagonal, heptagonal, octagonal, nonagonal, decagonal or star-like, cross-section.

Further, the second flexible tube may have an inner diameter of 10 mm to 60 mm, in particular 12 mm to 50 mm, preferably 15 mm to 40 mm. For example, if the medical device is provided for a treatment of an esophagus, the medical device may have an inner diameter of **11** mm. Alternatively, for example, if the medical device is provided for a treatment of another intracorporal site, the medical device may have an inner diameter of 15 mm.

Further, the second flexible tube may have a wall thickness, in particular film wall thickness, of 50 µm to 500 µm, in particular 60 µm to 350 µm, preferably 70 µm to 250 µm. For example, the second flexible tube may have a wall thickness of 80 µm.

Further, the second flexible tube may, in particular independently from the first flexible tube, comprise or consist of a polymer, in particular a natural polymer and/or synthetic polymer and/or in vivo degradable or resorbable polymer, in particular selected from group consisting of polyolefin, polyethylene, polypropylene, polyvinyl chloride, polytetrafluorethylene, polyurethane, polyether block amide, polycarbonate, ethylene vinyl acetate, polyester, polyacrylate, polyether ether ketone, polyethylene terephthalate, polyethylene terephthalate glycol, silicone, chitosan, cellulose, silk, gellan gum, polycaprolactone, polylactic acid, polyglycolide, polylactic-co-glycolic acid, polyvinyl alcohol, polyhydroxyalkanoate, poly-3-hydroxybutyrate, polytrimethylene carbonate, copolymers of at least two of the afore-said polymers and mixtures of at least two of the afore-said polymers.

Principally, the first flexible tube and the second flexible tube may be formed the same or differently, in particular in terms of length, inner diameter, wall thickness, cross-section, polymer or a combination of at least two of the afore-said properties.

Preferably, the second flexible tube may have a retractable, in particular foldable, or stretchable (i.e. expandable), in particular unfoldable, distal end portion, in particular having a length of 15 mm to 120 mm, in particular 30 mm to 100 mm, preferably 40 mm to 90 mm.

Further, the first flexible tube and/or second flexible tube may be manufactured independently from each other by means of die cut, radiofrequency welding, impulse welding, laser welding, ultrasonic welding, heating press welding, gluing, sewing or printing.

Further, both the first flexible tube and the second flexible tube may be provided by cutting a suitable tube material into a pattern and bonding longitudinal sides of the pattern, in particular by welding, for example with the aid of a sonotrode, to form a flexible tube.

Preferably, the second flexible tube is longer than the first flexible tube. For example, the second flexible tube may be 3 % to 60 %, in particular 7 % to 50 %, preferably 10 % to 40 %, longer than the first flexible tube. More specifically, the second flexible tube may be 1 cm to 25 cm, in particular 2 cm to 20 cm, preferably 3 cm to 18 cm, longer than the first flexible tube.

In an embodiment of the invention, the second flexible tube, in particular the distal end portion of the second flexible tube, overhangs or exceeds the distal end portion of the first flexible tube. This embodiment advantageously allows viewing of correct placement of the fluid drainage device into the patient's body.

In a further embodiment of the invention, the medical device further comprises a first connecting element and a second connecting element.

Preferably, the first connecting element is adapted to connect, in particular positive connect and/or frictionally connect and/or materially bond, preferably positive connect and/or frictionally connect, a proximal end portion of the endoscope to the proximal end portion of the first flexible tube.

More specifically, the first connecting element may be arranged or located at the proximal end portion of the first flexible tube. In addition, the first connecting element may be arranged or located at the proximal end portion of the second flexible tube. In particular, the first connecting element, in particular a distal end portion of the first connecting element, may be at least partially or sectionally, in particular only partially or sectionally or completely, inserted or insertable into the proximal end portion of the first flexible tube. In addition, the first connecting element, in particular a distal end portion of the first connecting element, may be at least partially or sectionally, in particular only partially or sectionally or completely, inserted or insertable into the proximal end portion of the second flexible tube.

More preferably, the first connecting element comprises or is in the form of a double-sleeve, preferably wherein one of the two sleeves, in particular a distal end portion of one of the two sleeves, is at least partially or sectionally, in particular only partially or sectionally or completely, inserted or insertable into the proximal end portion of the first flexible tube and the other of the two sleeves, in particular a distal end portion of the other of the two sleeves, is at least partially or sectionally, in particular only partially or sectionally or completely, inserted or insertable into the proximal end portion of the second flexible tube. Preferably, the double-sleeve is in the form of a one-piece construction.

Further, the first connecting element may be secured to the proximal end portion of the first flexible tube and optionally second flexible tube, in particular via a securing element such as a thread or wire being tied around the proximal end portion of the first flexible tube and optionally second flexible tube. With respect to further features and advantages in respect of the thread or wire, in particular regarding useful materials for the thread or wire, reference is made to the materials disclosed in the following in respect of the pulling element of the second flexible tube, which do apply mutatis mutandis.

Alternatively, the first connecting element may comprise a locking element, in particular a sleeve locker, being adapted to secure the first connecting element, in particular a distal end portion of the first connecting element, to the first flexible tube and optionally flexible tube, in particular to the proximal end portion of the first flexible tube and optionally second flexible tube. The locking element may be fixed to the first connecting element using a screw.

Preferably, the first connecting element has a hollow cylindrical shape or hollow conical shape. Further, the first connecting element may have an adjustable inner diameter. The inner diameter may be, for example, from 5 mm to 40 mm, in particular 8 mm to 30 mm, preferably 7 mm to 25 mm. In particular, the inner diameter may be adjustable via a locking or tightening member, such as a screw, in particular turning screw, or rotary knob. Thus, a connection between the proximal end portion of the endoscope and the proximal end portion of the first flexible tube and optionally second flexible tube may be conveniently achieved by narrowing the inner diameter of the first connecting element.

Further, the first connecting element may be manufactured by means of injection molding, 3D printing or CNC machining.

Further, the first connecting element may comprise or consist of a material selected from the group consisting of plastic material, in particular rigid plastic material, nylon, acrylonitrile butadiene styrene, polyoxymethylene homopolymer, polyoxymethylene copolymer, polyvinylchloride, polytetrafluorethylene, polylactide, polycarbonate, polypropylene, polyurethane, polyether ether ketone, polysulfone, metal such as aluminum and/or titanium, alloy such as steel, and mixtures of at least two of the afore-said materials.

Preferably, the second connecting element is adapted to connect, in particular positive connect and/or frictionally connect and/or materially bond, preferably materially bond, for example glue or weld, a distal end portion of the endoscope to the distal end portion of the second flexible tube or to a portion of the second flexible tube being arranged or located proximal to the distal end portion of the second flexible tube.

More specifically, the second connecting element may be arranged or located at the distal end portion of the second flexible tube or at a portion of the second flexible tube being located or arranged proximal to the distal end portion of the second flexible tube.

Preferably, the second connecting element comprises or is in the form of at least one adhesive tape, i.e. only one adhesive tape or a plurality of adhesive tapes, and/or at least one adhesive flap, i.e. only one adhesive flap or a plurality of adhesive flaps. For example, the second connecting element may comprise or be in the form of three adhesive tapes, preferably wherein one of the three adhesive tapes is arranged, in particular placed, on a connector for the endoscope, wherein said connector is arranged, in particular welded, on the second flexible tube, and the remaining two of the three adhesive tapes are arranged, in particular placed, on two flaps of the second flexible tube.

Further, the at least one adhesive tape may be in the form of at least one stripe, in particular at least one square-shaped stripe, for example having a dimension of 20 mm x 20 mm.

In general, the first connecting element and/or second connecting element may be adapted to connect the proximal end portion and distal end portion, respectively of an endoscope having a size, i.e. diameter, of 5 mm to 15 mm.

In a further embodiment of the invention, the medical device further comprises a manipulating element. Preferably, the manipulating element is connected to the distal end portion of the second flexible tube. Further preferably, the manipulating element is adapted to retract, in particular fold back or roll up, or stretch, in particular unfold or unroll, the distal end portion of the second flexible tube. Thus, a blocking of a camera of the endoscope may be advantageously circumvented.

Further, the manipulating element may be inserted via a working channel of the endoscope.

Further, the manipulating element may in particular comprise or consist of a semi rigid material. With respect to useful semi rigid materials, reference is made in its entirety to the following description.

In a further embodiment of the invention, the manipulating element is in the form of a longitudinal element, in particular in the form of a thread or wire. More specifically, the thread may be in the form of a monofilament, pseudo monofilament or multifilament, in particular braided or twisted multifilament. Further, the thread may have a diameter of USP 0, i.e. 0.350 mm to 0.399 mm or of USP 3/0, i.e. 0.200 mm to 0.249 mm. Further, the thread may comprise or consist of a polymer, in particular selected from the group consisting of polyester such as polyethylene terephthalate, polyolefin such as polyethylene and/or polypropylene, cotton and mixtures of at least two of the afore-said polymers. Preferably, the thread comprises or consists of a polyester, in particular polyethylene terephthalate. The wire may comprise or consist of a metal or alloy, in particular steel, and mixtures thereof.

Preferably, the manipulating element is attached or connected, in particular sewn, stitched or knotted, to the distal end portion, in particular along a longitudinal side of the distal end portion, of the second flexible tube. Further, the manipulating element is preferably secured to the distal end portion of the second flexible tube, in particular by means of at least one knot, i.e. only one knot or a plurality of knots.

In a further embodiment of the invention, a distal end portion of the manipulating element has at least one mark, i.e. only one mark or a plurality of marks. The marks may be in the form of at least two marks, in particular two horizontal marks around the distal end portion of the manipulating element. The at least one mark is preferably visible through a camera of the endoscope. Advantageously, the at least one mark preferably indicates a user when the second flexible tube is extended. The at least one mark may be generated by laser printing, ink printing, machining, or over-molding technologies. Further, the at least one mark may have a width from 0.1 mm to 10 mm, in particular from 0.2 mm to 8 mm, preferably from 0.3 mm to 5 mm.

In a further embodiment of the invention, the medical device further comprises a pulling element. Preferably, the pulling element is adapted to fold the distal end portion of the second flexible tube, in particular in the direction or towards the proximal end portion of the second flexible tube. Thus, avoidance of blocking of a camera of the endoscope may be advantageously further optimized.

Preferably, the pulling element is attached or connected, in particular sewn, stitched or knotted, to the distal end portion, in particular to a middle of the distal end portion or along a longitudinal side of the distal end portion, of the second flexible tube. Further, the pulling element is preferably secured to the distal end portion, in particular to a middle of the distal end portion or along a longitudinal side of the distal end portion, of the second flexible tube, in particular by means of at least one knot, i.e. only one knot or a plurality of knots, for example two or more knots.

Further, the pulling element may be arranged or located outside the first flexible tube and/or second flexible tube. Alternatively, the pulling element may be arranged or located at least sectionally, in particular only sectionally, inside the second flexible tube and in particular up to the proximal end portion of the second flexible tube.

Further, the pulling element may be in the form of a wire, in particular plastic wire or metal wire. In particular, the pulling element may comprise or consist of a material selected from the group consisting of polyester, polyethylene terephthalate, polyolefin such as polyethylene and/or polypropylene, cotton, alloy, steel and combinations, in particular mixtures, of at least two of the afore-said materials.

In a further embodiment of the invention, the medical device further comprises a sheath being adapted to surround or encase the fluid drainage device or surrounding the fluid drainage device at least partially or sectionally, in particular only partially or sectionally or completely.

In a further embodiment of the invention, the medical device is free of a sheath being adapted to surround or encase the fluid drainage device or surrounding the fluid drainage device at least partially or sectionally, in particular only partially or sectionally or completely.

In a further embodiment of the invention, the medical device further comprises a third connecting element. The third connecting element is preferably arranged or located at the distal end portion of the second flexible tube. Preferably, the third connecting element is adapted to connect, in particular positive connect and/or frictionally connect and/or materially bond, a distal end portion of a/the manipulating element to the distal end portion of the second flexible tube and in particular to disconnect the distal end portion of the manipulating element from the distal end portion of the second flexible tube. For example, the third connecting element may be in the form of a screw. Thus, the distal end portion of the second flexible tube may be advantageously retracted, folded or deployed via the manipulating element. The third connecting element is preferably attached, in particular materially bonded, for example glued or welded, and/or via a thread, to the distal end portion of the second flexible tube. With respect to further features and advantages of the thread, reference is made to the respective features and advantages disclosed in terms of the thread as an embodiment of the manipulating element which do apply mutatis mutandis.

In a further embodiment of the invention, the medical device or the second flexible tube further comprises an insertion guiding element. The insertion guiding element is arranged or located at the proximal end portion of the second flexible tube. More specifically, the insertion guiding element, in particular a distal end portion of the insertion guiding element, may be at least partially or sectionally, in particular only partially or sectionally or completely, inserted into the proximal end portion of the second flexible tube. Further, the insertion guiding element may be secured to the proximal end portion of the second flexible tube, in particular via a securing element such as a thread or wire being tied around the proximal end portion of the second flexible tube. With respect to further features and advantages in respect of the thread and the wire, reference is made to the respective features and advantages disclosed in terms of the thread or wire as an embodiment of the manipulating element or pulling element of the medical device which do apply mutatis mutandis.

Preferably, the insertion guiding element is adapted to push and/or insert the fluid drainage device, in particular fluid drainage device being at least partially or sectionally, in particular only partially or sectionally or completely, surrounded by a sheath, into and/or through the second flexible tube.

For example, the insertion guiding element may have a hollow cylindrical shape or a hollow conical shape. Further, the insertion guiding element may have an inner diameter of 5 mm to 35 mm, in particular 10 mm to 30 mm, preferably 12 mm to 25 mm.

Further, the insertion guiding element may be manufactured by means of injection molding, 3D printing or CNC machining.

Further, the insertion guiding element may comprise or consist of a material selected from the group consisting of plastic material, in particular rigid plastic material, nylon, acrylonitrile butadiene styrene, polyoxymethylene homopolymer, polyoxymethylene copolymer, polyvinylchloride, polytetrafluorethylene, polylactide, polycarbonate, polypropylene, polyurethane, polyether ether ketone, polysulfone, metal such as aluminum and/or titanium, alloy such as steel, and mixtures of at least two of the afore-said materials.

In a further embodiment of the invention, the insertion guiding element is adapted to be connected, in particular positively connected and/or frictionally connected and/or materially bonded, or is connected, in particular positively connected and/or frictionally connected and/or materially bonded, to the fluid drainage device, in particular to a proximal end portion of the fluid drainage device, preferably to a proximal end portion of a fluid communication element of the fluid drainage device. Thus, stability of the fluid drainage device may be advantageously maintained during insertion and in particular it may be kept as one element during insertion maneuvers. With respect to further features and advantages of the fluid communication element, reference is made in its entirety to the following description.

In a further embodiment of the invention, a handle of the medical device is adapted to be connected, in particular positively connected and/or frictionally connected and/or materially bonded, or is connected, in particular positively connected and/or frictionally connected and/or materially bonded, to the fluid drainage device, in particular to a proximal end portion of the fluid drainage device, preferably to a proximal end portion of a fluid communication element of the fluid drainage device. Thus, stability of the fluid drainage device may be advantageously maintained during insertion and in particular it may be kept as one element during insertion maneuvers. The handle may be part of the insertion guiding element. In particular, the handle and the insertion guiding element may be in the form of a one-piece construction. Further, the handle may be attached to the insertion guiding element or may be pushed onto the insertion guiding element. Alternatively, the handle may be in the form of a separate element of the medical device, in particular being independent from the insertion guiding element. Further, the handle is preferably arranged or located at or onto a proximal end portion of the insertion guiding element. With respect to further features and advantages of the fluid communication element, reference is made in its entirety to the following description.

In a further embodiment of the invention, the first connecting element and the insertion guiding element are formed together in one piece, i.e. are together in the form of a one-piece construction. Preferably, a distal end portion of the first connecting element is inserted into or inside the proximal end portion of the first flexible tube and a distal end portion of the insertion guiding element is inserted into or inside the proximal end portion of the second flexible tube. With respect to further features and advantages, in particular in terms of the first connecting element and the insertion guiding element in this embodiment, reference is made in its entirety to the previous description.

The invention as claimed refers to a medical kit, in particular for carrying out an intracorporal, preferably endoluminal and/or endocavitary, vacuum therapy, preferably for draining pathological body fluids, preferably from intracorporal cavities such as esophagus, colon or fistula.

The medical kit comprises, in particular spatially separated from each other, at least the following components:
- a medical device as specified in claim 1 and
- a fluid drainage device being adapted to drain pathological body fluids and being at least partially or sectionally surrounded by a sheath.

Preferably, the sheath may have a tubular shape, in particular a regular or irregular tubular shape. More preferably, the sheath may be in the form of a capsule.

Preferably, a proximal end portion of the sheath is open and a distal end portion of the sheath is closed.

Further, the sheath may have a length of 10 mm to 250 mm, in particular 20 mm to 200 mm, preferably 40 mm to 180 mm. For example, the sheath may have a length of 130 mm.

Further, the sheath may have an inner diameter of 5 mm to 120 mm, in particular 6 mm to 100 mm, preferably 8 mm to 90 mm. For example, the sheath may have a diameter of 25 mm.

Further, the sheath may have a wall thickness of 30 µm to 500 µm, in particular 50 µm to 400 µm, preferably 60 µm to 350 µm. For example, the sheath may have a wall thickness of 80 µm.

Further, the sheath may comprise or consist of a polymer, in particular selected from the group consisting of a polymer, in particular a natural polymer and/or synthetic polymer and/or in vivo degradable or resorbable polymer, in particular selected from group consisting of polyolefin, polyethylene, polypropylene, polyvinyl chloride, polytetrafluorethylene, polyurethane, polyether block amide, polycarbonate, ethylene vinyl acetate, polyester, polyacrylate, polyether ether ketone, polyethylene terephthalate, polyethylene terephthalate glycol, silicone, chitosan, cellulose, silk, gellan gum, polycaprolactone, polylactic acid, polyglycolide, polylactic-co-glycolic acid, polyvinyl alcohol, polyhydroxyalkanoate, poly-3-hydroxybutyrate, polytrimethylene carbonate, copolymers of at least two of the afore-said polymers and mixtures of at least two of the afore-said polymers.

Principally, the sheath may be formed the same way as the first flexible tube and/or second flexible tube of the medical device according to the first aspect of the present invention. However, it is preferred if the sheath is formed differently like the first flexible tube and/or second flexible tube of the medical device according to the first aspect of the present invention.

Alternatively, in embodiments not forming part of the invention as claimed, the medical kit may be free of the sheath.

In a further embodiment of the disclosure, the fluid drainage device comprises a fluid collection element and a fluid communication element. The fluid collection element is preferably adapted to collect, in particular absorb or soak, body fluids, in particular pathological body fluids.

Principally, the fluid collection element may have a textile structure or be in the form of a textile structure. For example, the fluid collection element may have a mesh structure, in particular rolled mesh structure, or be in the form of a mesh structure, in particular rolled mesh structure, for example comprising or consisting of a polyolefin, preferably polypropylene.

Alternatively, the fluid collection element may have a grid or grid-like structure or may be in the form of a grid or grid-like structure.

Further, the fluid collection element may comprise flock fibers. In other words, the fluid collection element may be in the form of a flocked element. As regards useful materials for the flock fibers, reference is made to the polymers disclosed in the following in respect of the fluid collection element.

Further, the fluid collection element may have a ceramic structure or be in the form of a ceramic structure.

Preferably, the fluid collection element has a foam or sponge structure or is in the form of a foam or sponge.

Further, the fluid collection element may preferably have a porous structure.

More specifically, the fluid collection element may comprise a linear pore density of 3 pores per centimeter to 30 pores per centimeter, in particular 3 pores per centimeter to 25 pores per centimeter, preferably 3 pores per centimeter to 20 pores per centimeter.

The term "linear pore density" as used according to the present invention refers to pores which are arranged successively, i.e. in a row.

Especially preferably, the fluid collection element has an open-cell or open-pored foam structure or open-cell or open-pored sponge structure or is in the form of an open-cell or open-pored foam or open-cell or open-pored sponge.

Further, the fluid collection element may be of any shape which allows placement of the fluid drainage device into the patient's body.

More specifically, the fluid collection element may have a polyhedral or non-polyhedral shape. For example, the fluid collection element may be in the form of a cube, cuboid, prism, cylinder, pyramid, cone or ball.

Further, the fluid collection element may have a cornerless, i.e. round, in particular circular, oval or elliptic, cross-section. Alternatively, the fluid collection element may have a non-round, in particular polygonal, for example triangular, rectangular, square, trapezoid, rhomboid, pentagonal, hexagonal, heptagonal, octagonal, nonagonal, decagonal or star-like, cross-section.

Preferably, the fluid collection element has a cylindrical shape or is in the form of a cylinder.

Further, the fluid collection element may comprise a canal, in particular an inner canal. Preferably, the canal extends along, i.e. in a longitudinal direction, of the fluid collection element. The canal may have an inner diameter from 0.5 mm to 15 mm, in particular 1 mm to 5 mm, preferably 1 mm to 4 mm. The canal of the fluid collection element may have a cornerless, i.e. round, cross-section, for example a circular, oval or elliptical cross-section. Alternatively, the canal of the fluid collection element may have a non-round, in particular polygonal, for example triangular, rectangular, square, trapezoid, rhomboid, pentagonal, hexagonal, heptagonal, octagonal, nonagonal, decagonal or star-like cross-section.

Further, the fluid collection element may comprise a longitudinal cut, in particular made by means of a punch or a cross-section die. In particular, the longitudinal cut can be in the form of a slot.

Further, the fluid collection element may have an outer diameter from 3 mm to 50 mm, in particular 5 mm to 40 mm, preferably 8 mm to 35 mm.

Further, the fluid collection element, in particular the canal of the fluid collection element, may have a length of 10 mm to 250 mm, in particular 50 mm to 200 mm, preferably 80 mm to 150 mm.

Further, the fluid collection element may surround or engird, in particular at least partially or sectionally, preferably only partially or sectionally, the fluid communication element. Preferably, the fluid collection element only surrounds or engirds a distal end portion of the fluid communication element.

Further, the fluid collection element is preferably connected to the fluid communication element, in particular at least partially or sectionally, preferably only partially or sectionally. Especially preferably, the fluid collection element is only connected to the distal end portion of the fluid communication element.

Further, the fluid collection element may be connected positively and/or frictionally to the fluid communication element. For example, the fluid collection element may be connected to the fluid communication element via a connecting element, in particular being configured for form-fit and/or frictionally connecting the fluid collection element to the fluid communication element, in particular to the distal end portion of the fluid communication element. The connecting element may be selected from the group consisting of a thread, a wire or a clip. Further, the connecting element may penetrate through the fluid collection element. Further, the connecting element may be secured, in particular by at least one knot, to the fluid collection element. Further, the connecting element may preferably constrict the fluid collection element, at least partially or sectionally.

Further, the connecting element may engage with holes and/or perforations of the fluid communication element, in particular of the distal end portion of the fluid communication element. With respect to further features and advantages of the connecting element, in particular in the form of a thread or wire, reference is made to the respective features and advantages disclosed in terms of the pulling element of the second flexible tube of the medical device according to the first aspect of the present invention.

Alternatively or in combination, the fluid collection element may be connected to the fluid communication element, in particular to the distal end portion of the fluid communication element, by a material bond. For example, the fluid collection element may be connected to the fluid communication element, in particular to the distal end portion of the fluid communication element, by means of gluing and/or welding, in particular vibration welding or thermal welding.

Further, the fluid collection element may comprise or consist of an in vivo degradable or in vivo resorbable polymer, in particular in vivo degradable or in vivo resorbable natural polymer and/or in vivo degradable or in vivo resorbable synthetic polymer.

The in vivo degradable or in vivo resorbable polymer may be selected from the group consisting of polyvinyl alcohol, polyhydroxyalkanoate, polyglycolide, polylactide, polydioxanone, poly-3-hydroxybutyrate, poly-4-hydroxybutyrate, polytrimethylene carbonate, poly-ε-caprolactone, polyethylene glycol, collagen, hyaluronic acid, polyglutamic acid, gellan gum, gelatin, fibrin, alginate, copolymers of at least two of the afore-said polymers such as poly(lactide-co-glycolide), and mixtures of at least two of the afore-said polymers.

Alternatively, the fluid collection element may comprise or consist of an in vivo non-degradable or in vivo non-resorbable polymer, in particular in vivo non-degradable or in vivo non-resorbable natural polymer and/or in vivo non-degradable or in vivo non-resorbable synthetic polymer. The in vivo non-degradable or in vivo non-resorbable polymer may be selected from the group consisting of polyolefin, polyester, polyamide, polyurethane, elastomer such as thermoplastic elastomer, polyetherketone, organic polysulfide, silicone, cellulose, silk, chitosan, copolymers of at least two of the afore-said polymers, and mixtures of at least two of the afore-said polymers.

The polyolefin may be selected from the group consisting of polyethylene, low-density polyethylene, high-density polyethylene, high-molecular-weight polyethylene, ultra-high-molecular-weight polyethylene, polypropylene, polytetrafluorethylene, polyvinylidene fluoride, polyvinylidene chloride, polytetrafluorpropylene, polyhexafluorpropylene, copolymers of at least two of the afore-said polyolefins and mixtures of at least two of the afore-said polyolefins.

The polyester may be selected from the group consisting of polyethylene terephthalate, polypropylene terephthalate, polybutylene terephthalate, copolymers of at least two of the afore-said polyesters and mixtures of at least two of the afore-said polyesters.

The polyamide may be selected from the group consisting of polyamide 6 (polymer of ε-caprolactam or ω-aminocaproic acid units), polyamide 66 (polymer of hexamethylenediamine units and adipic acid units), polyamide 69 (polymer of hexamethylenediamine units and azelaic acid units), polyamide 612 (polymer of hexamethylenediamine units and dodecandioic acid units), polyamide **11** (polymer of 11-aminoundecanoic acid units), polyamide 12 (polymer of laurolactam acid units and ω-aminododecanoic acid units), polyamide 46 (polymer of tetramethylenediamine units and adipic acid units), polyamide 1212 (polymer of dodecandiamine units and dodecandioic acid units), polyamide 6/12 (polymer of caprolactam units and laurolactam units), polyamide 66/610 (polymer of hexamethylenediamine units, adipic acid units and sebacic acid units), copolymers of at least two of the afore-said polyamides and mixtures of at least two of the afore-said polyamides.

The polyurethane may be selected from the group consisting of aliphatic polycarbonate urethane, silicone polycarbonate urethane, polyether urethane, siliconepolyether urethane, polyurethane ether, copolymers of at least two of the afore-said polyurethanes and mixtures of at least two of the afore-said polyurethanes.

The thermoplastic elastomer may be selected from the group consisting of thermoplastic copolyamide, thermoplastic polyester elastomer, thermoplastic copolyester, thermoplastic elastomer on olefin basis, styrene block copolymer, thermoplastic elastomer on urethane basis, cross-linked thermoplastic elastomer on olefin basis, copolymers of at least two of the afore-said elastomers and mixtures of at least two of the afore-said elastomers.

The polyetherketone may be selected from the group consisting of polyetherketoneketone, polyetheretheretherketone, polyetheretherketoneketone, polyetherketoneetherketoneketone, copolymers of at least two of the afore-said polyetherketones and mixtures of at least two of the afore-said polyetherketones.

The fluid communication element may have a tubular shape, i.e. may be in the form of a tube, in particular plastic tube, preferably flexible plastic tube. More preferably, the fluid communication element is in the form of a drainage tube, in particular a redon drainage tube.

Further, the fluid communication element may have a cornerless, i.e. round, cross-section, in particular a circular, oval or elliptic cross-section.

Alternatively, the fluid communication element may have a non-round, in particular polygonal, cross-section. For example, the fluid communication element may have a triangular, rectangular, square, trapezoid, rhomboid, pentagonal, hexagonal, heptagonal, octagonal, nonagonal, decagonal or star-like cross-section.

Further, the fluid communication element may have a length from 200 mm to 1.500 mm, in particular 300 mm to 1.300 mm, preferably 350 mm to 1.000 mm.

Further, the fluid communication element may have an outer diameter of 0.5 mm to 15 mm, in particular 1 mm to 8 mm, preferably 1 mm to 5 mm.

Further, the fluid communication element may have an inner diameter of 0.1 mm to 13 mm, in particular 0.2 mm to 4 mm, preferably 0.5 mm to 2 mm.

Further, the fluid communication element may comprise or consist of a polymer, in particular a natural polymer and/or synthetic polymer and/or in vivo degradable or resorbable polymer, in particular selected from group consisting of polyolefin, polyethylene, polypropylene, polyvinyl chloride, polytetrafluorethylene, polyurethane, polyether block amide, polycarbonate, ethylene vinyl acetate, polyester, polyacrylate, polyether ether ketone, polyethylene terephthalate, polyethylene terephthalate glycol, silicone, chitosan, cellulose, silk, gellan gum, polycaprolactone, polylactic acid, polyglycolide, polylactic-co-glycolic acid, polyvinyl alcohol, polyhydroxyalkanoate, poly-3-hydroxybutyrate, polytrimethylene carbonate, copolymers of at least two of the afore-said polymers and mixtures of at least two of the afore-said polymers.

Further, the fluid communication element may be arranged or located, in particular at least partially or sectionally, preferably only partially or sectionally, inside the fluid collection element, preferably inside an inner canal of the fluid collection element.

More preferably, a distal end portion, in particular only a distal end portion, of the fluid communication element is, in particular at least partially or sectionally, preferably only partially or sectionally or completely, arranged inside the fluid collection element, preferably inside an inner canal of the fluid collection element. In other words, more preferably, a distal end portion, in particular only a distal end portion, of the fluid communication element is, in particular at least partially or sectionally, preferably only partially or sectionally or completely, surrounded or engirded by the fluid collection element. For example, the distal end portion of the fluid communication element being surrounded or engirded by the fluid collection element may have a length from 10 mm to 250 mm, in particular 40 mm to 200 mm, preferably 40 mm to 150 mm.

Further, the fluid communication element, in particular the distal end portion of the fluid communication element, may comprise holes and/or perforations. Expediently, the holes and/or perforations may be configured in the form or breakthroughs, i.e. as holes and/or perforations breaking through a wall of the fluid communication element. The holes and/or perforations are advantageously adapted to transfer the pathological body fluids, which have been taken up by the fluid collection element, into the fluid communication element. Preferably, the holes and/or perforations are arranged along, i.e. in a longitudinal direction, of the fluid communication element, in particular along the distal end portion of the fluid communication element. The holes and/or perforations may have, for example, a mutual distance of 1 mm to 20 mm, in particular 2 mm to 10 mm, preferably 3 mm to 8 mm. Further, the holes and/or perforations may have a cornerless, i.e. round, in particular circular, oval or elliptical, cross-section. Alternatively or in combination, the holes and/or perforations may have a non-round, in particular polygonal, for example triangular, square, rectangular, trapezoid, rhomboid, pentagonal, hexagonal, heptagonal, octagonal, nonagonal, decagonal or star-like, cross-section. Further, the holes and/or perforations may have a diameter of 0.1 mm to 5 mm, in particular 0.3 mm to 4 mm, preferably 0.5 mm to 3 mm.

Further, the fluid communication element may comprise a connector, in particular a luer-lock connector, preferably a male luer-lock connector. Preferably, the connector is in the form of a negative pressure or vacuum connector, i.e. a connector being adapted to connect a proximal end portion of the fluid communication element to an extension tube and/or to a negative pressure or vacuum source, in particular digital negative pressure or vacuum source. The negative pressure and vacuum source, respectively, may be, for example, in the form of a negative pressure and vacuum pump, respectively. More preferably, the connector is arranged at, in particular attached to, a proximal end portion of the fluid communication element.

The term "extension tube" as used according to the present invention refers to a tube being adapted to connect the fluid communication element to a negative pressure or vacuum source, in particular to a digital negative pressure or vacuum source.

Preferably, the sheath is adapted to surround or encase, in particular only, the fluid collection element of the fluid drainage device or surrounds or encases, in particular only, the fluid collection element of the fluid drainage device.

Further, the sheath is preferably in the form of a flexible, in particular compressible or pliant, sheath.

Further, the sheath may have a removable rigid or semi rigid interface, in particular being adapted to ease insertion of the fluid drainage device or of the fluid collection element of the fluid drainage device into the sheath.

Preferably, the sheath, in particular in a stretched, preferably unfolded or unrolled, condition, has an inner diameter being smaller than an outer diameter, in particular maximum outer diameter, of the fluid drainage device or of the fluid collection element of the fluid drainage device. Thus, the sheath is preferably adapted to compress the fluid drainage device or the fluid collection element of the fluid drainage device when being inserted into the sheath.

In a further embodiment of the invention, the sheath has a release element being adapted to release the fluid drainage device or the fluid collection element of the fluid drainage device from the sheath. The release element may comprise or be in particular in the form of an openable closure element, preferably in the form of a zip mechanism. Expediently, the release element is located at a distal end portion of the sheath.

Preferably, the release element extends axially, in particular along at least one longitudinal side, preferably along two oppositely arranged longitudinal sides, of the sheath, in particular of the distal end portion of the sheath.

Preferably, the release element comprises, in particular additionally comprises, or is in the form of a longitudinal element, in particular thread or wire, being connected, in particular sewn, i.e. stitched, to the distal end portion of the sheath. Thus, the fluid drainage device or fluid collection element of the fluid drainage device may be advantageously released from the sheath by pulling the longitudinal element, and thus undoing the connection, in particular sewing, stitching or clipping, of the longitudinal element to the sheath. With respect to further features and advantages of the thread or wire, reference is made to the respective features and advantages disclosed in terms of the thread or wire as an embodiment of the pulling element of the second flexible tube of the medical device according to the first aspect of the invention.

Alternatively or in combination, the release element may comprise or may be in the form of a number of material weakenings of the sheath. For example, the release element may comprise or may be in the form of a perforation or material thinning.

Further, the release element may extend linear or non-linear, in particular zigzag or undulating, along at least one longitudinal side, preferably along two oppositely arranged longitudinal sides, of the sheath, in particular of the distal end portion of the sheath. For example, the release element may extend over a length of 10 mm to 100 mm, in particular 20 mm to 90 mm, preferably 30 mm to 80 mm, along at least one longitudinal side, in particular along two oppositely arranged longitudinal sides, of the sheath, in particular of the distal end portion of the sheath. If the medical device is provided for the vacuum therapy of an esophagus, the release element may, for example, extend over a length of 50 mm along at least one longitudinal side, in particular along two oppositely arranged longitudinal sides, of the sheath, in particular of the distal end portion of the sheath. Alternatively, if the medical device is provided for another intracorporal site, the release element may, for example, extend over a length of 70 mm along at least one longitudinal side, in particular along two oppositely arranged longitudinal sides, of the sheath, in particular of the distal end portion of the sheath.

In a further embodiment of the invention, a tail, in particular longitudinal tail, is formed at a proximal end portion of the sheath. Preferably, the tail is adapted to remove the sheath from the inside of the patient's body after release of the fluid drainage device or fluid collection element of the fluid drainage device from the sheath. The tail may be, for example, in the form of a stripe. Further, the tail may have a length of 50 mm to 1.500 mm, in particular 150 mm to 1.200 mm, preferably 250 mm to 1.000 mm. For example, if the medical kit is provided for the wound therapy of an esophagus, the tail may have a length of 650 mm. Alternatively, for example, if the medical device is provided for a wound therapy of another intracorporal site, the tail may have a length of 310 mm. Further, the tail may have a width of 1 mm to 50 mm, in particular 5 mm to 40 mm, preferably 10 mm to 30 mm. For example, the tail may have a width of 20 mm. Further, the tail may comprise or consist of a polymer, in particular a natural polymer and/or synthetic polymer and/or in vivo degradable or resorbable polymer, in particular selected from group consisting of polyolefin, polyethylene, polypropylene, polyvinyl chloride, polytetrafluorethylene, polyurethane, polyether block amide, polycarbonate, ethylene vinyl acetate, polyester, polyacrylate, polyether ether ketone, polyethylene terephthalate, polyethylene terephthalate glycol, silicone, chitosan, cellulose, silk, gellan gum, polycaprolactone, polylactic acid, polyglycolide, polylactic-co-glycolic acid, polyvinyl alcohol, polyhydroxyalkanoate, poly-3-hydroxybutyrate, polytrimethylene carbonate, copolymers of at least two of the afore-said polymers and mixtures of at least two of the afore-said polymers. Principally, in terms of polymers, the tail and the sheath may be formed the same or differently.

In a further embodiment of the invention, the sheath has a marking element being adapted to indicate a proximal beginning or start of the release element. The marking may be, for example, in the form of a colored, in particular red, marking.

In a further embodiment of the invention, the medical kit comprises at least one further kit component selected from the group consisting of feeding element, manipulating element, pulling element, at least one pushing element, clamp, endoscope, syringe, dilatator extensor tube, tube connectors such as luer lock connector and/or Y-connector, connector being adapted to be arranged between the fluid communication element and a/the dilatator extensor tube, lubricant, wipes, scissors, instructions for use and combinations of at least two of the afore-said further kit components.

The feeding element may be in particular in the form of a two-piece component, i.e. a component comprising or consisting of two pieces. More specifically, the feeding element may comprise a first piece having at least partially or sectionally a cylindrical form and/or conical form. Further, the first piece may have an external thread, in particular between a proximal end portion and a distal end portion of the first piece. Expediently, the feeding element may additionally comprise a second piece, in particular in the form of a cap nut or union nut, having an internal thread being complementary to the external thread of the first piece.

The feeding element is adapted to insert the fluid drainage device or the fluid collection element of the fluid drainage device into the sheath. Preferably, the feeding element has an internal diameter being smaller than an outer diameter, in particular maximum outer diameter, of the fluid drainage device or fluid collection element of the fluid drainage device. Thus, the fluid drainage device or fluid collection element may be compressed and pushed through the feeding element until the fluid drainage device or fluid collection element is completely placed inside the sheath.

Further, the feeding element, in particular the above-mentioned first piece and second piece of the feeding element, may be, in particular independent from each other, manufactured by means of injection molding, 3D printing or CNC machining.

Further, a distal end portion of the feeding element, in particular the distal end portion of the above-mentioned first piece of the feeding element, is preferably adapted to be inserted into the sheath. The sheath may be fixed to the feeding element, in particular via a positive or form-locking connection and/or a frictional connection and/or a material connection. For example, the sheath may be fixed to the feeding element by means of a screw or screwing mechanism.

Further, the feeding element, in particular the above-mentioned first piece and/or second piece, may, in particular independent from each other, comprise or consist of a material selected from the group consisting of a rigid plastic material, nylon, acrylonitrile butadiene styrene, polyoxymethylene homopolymer, polyoxymethylene copolymer, polyvinylchloride, polytetrafluorethylene, polycarbonate, polypropylene, polyurethane, polyether ether ketone, polysulfone, metal such as aluminum and/or titanium, alloy such as steel, and mixtures of at least two of the afore-said materials.

Further, the feeding element, in particular the above-mentioned first piece and/or second piece, may be in the form of a rigid component.

Further, the sheath may be manufactured by means of die cut, radiofrequency welding, laser welding, impulse welding, ultra-sound welding, heating press welding, gluing, sewing or printing.

Further, the manipulating element may be adapted to be passed through a work channel of the endoscope. Preferably, the manipulating element is connected to the distal end portion of the second flexible tube. The connection may be, for example, achieved via a screw or a clipping element. The function of the manipulating element is advantageously to retract and in particular deploy the distal end portion of the second flexible tube. The manipulating element may be, for example, in the form of a wire such as a plastic or metallic wire.

Further, the manipulating element may have one or several markings, in particular at its distal end portion. The markings may be in the form of at least two marks, in particular two horizontal marks around the manipulating element, in particular around the distal end portion of the manipulating element. The marks are preferably visible through the endoscope camera and indicate the user when the second flexible tube is extended. The marks may be created by laser printing, ink printing, machining, or over-molding technologies. Further, the marks may have a width from 0.1 mm to 10 mm, in particular from 0.2 mm to 8 mm, preferably from 0.3 mm to 5 mm.

With respect to further features and advantages regarding the manipulating element, reference is made in its entirety to the respective features and advantages disclosed under the first aspect of the present invention which do apply mutatis mutandis.

With respect to further features and advantages regarding the pulling element, reference is made in its entirety to the respective features and advantages disclosed under the first aspect of the present invention which do apply mutatis mutandis.

The at least one pushing element may be adapted to push the fluid drainage device or the fluid collection element of the fluid drainage device, in particular being inside the sheath, through the second flexible tube of the medical device according to the first aspect of the invention. In particular, the at least one pushing element may be in the form of at least one flexible hollow tube. This advantageously allows the fluid communication element of the fluid drainage device to pass through a lumen of the at least one pushing element. Expediently, the at least one pushing element may have a length being greater than the length of the second flexible tube of the medical device according to the first aspect of the invention. Further, the at least one pushing element may have a marking, in particular close to a proximal end portion of the at least one pushing element. Thus, if said marking arrives at the insertion guiding element of the second flexible tube of the medical device according to the first aspect of the invention, the user knows that the distal end portion of the pushing element has reached the distal end portion of the second flexible tube, and thus the fluid drainage device or the fluid collection element of the fluid drainage device, in particular being inside the sheath, have left the second flexible tube. Further, the at least one pushing element may have a handle or may be free of a handle.

Further, the at least one pushing element may be in the form of a first pushing element and a second pushing element. With respect to features and advantages of the first pushing element and the second pushing element, reference is made in its entirety to the preceding paragraph referring to the at least one pushing element, however, preferably with the proviso that the second pushing element is shorter than the first pushing element. Preferably, the first pushing element is adapted to push the fluid drainage device or the fluid collection element of the fluid drainage device, in particular being inside the sheath, through the second flexible tube of the medical device according to the first aspect of the invention. The second pushing element is preferably adapted to push the fluid drainage device or the fluid collection element of the fluid drainage device through the feeding element until the fluid drainage device or the fluid collection element of the fluid drainage device reaches the sheath.

Further, the at least one pushing element, in particular the first pushing element and the second pushing element, may be, in particular independent from each other, manufactured by means of extrusion, cut to length or printing.

Further, the at least one pushing element, in particular the first pushing element and the second pushing element, may, in particular independent from each other, comprise or consist of a material selected from the group consisting of plastic material, polyphenylsulfone, polyamide, polyurethane, polyolefin, polyethylene, polytetrafluorethylene, polypropylene, polyvinylchloride, polycarbonate, and mixtures of at least two of the afore-said materials.

Further, the clamp may be in the form of a plastic clamp, in particular being adapted to hold the fluid communication element when the fluid collection element of the fluid drainage device and in particular the sheath is fitted inside the at least one pushing element.

With respect to further features and advantages of the kit, in particular in terms of the medical device according to the first aspect of the present invention, reference is made in its entirety to the embodiments disclosed under the first aspect of the present invention, which do apply mutatis mutandis.

According to a third aspect, the present disclosure refers to a sheath. The sheath is adapted to surround or encase a fluid drainage device at least partially or sectionally, in particular only partially or sectionally or completely.

With respect to further features and advantages of the sheath, reference is made in its entirety to the previous description, in particular to the respective embodiments disclosed under the second aspect of the present invention, which do apply mutatis mutandis.

According to a fourth aspect, the present invention refers to a further medical kit, in particular for carrying out an intracorporal, preferably endoluminal and/or endocavitary, vacuum therapy, preferably for draining body fluids, preferably from intracorporal cavities, for example located in the esophagus, stomach or colon or for example a fistula such as anal fistula.

The further medical kit comprises, in particular spatially separated from each other, at least the following components:
- a fluid drainage device being adapted to drain body fluids and
- a sheath being adapted to surround or encase the fluid drainage device at least partially or sectionally, in particular only partially or sectionally or completely, or a sheath surrounding or encasing the fluid drainage device at least partially or sectionally, in particular only partially or sectionally or completely.

The further medical kit may additionally comprise at least one further kit component selected from the group consisting of medical device according to the first aspect of the invention, feeding element, manipulating element, pulling element, at least one pushing element, clamp, an endoscope, syringe, dilatator extensor tube, tube connectors such as luer lock connector and/or Y-connector, connector being adapted to be arranged between the fluid communication element and a/the dilatator extensor tube, lubricant, wipes, scissors, instructions for use and combinations of at least two of the afore-said further kit components.

With respect to further features and advantages of the further medical kit, reference is made in its entirety to the previous description, in particular to the respective embodiments disclosed under the first and second aspect of the present invention, which do apply mutatis mutandis.

According to a fifth aspect, not forming part of the invention as claimed, the present disclosure refers to a method for treating an inner body cavity of a human or non-human mammal, preferably by means of a vacuum therapy, more preferably by means of an endoscope-assisted procedure.

The inner body cavity may be, for example, selected from the group consisting of esophagus, stomach, colon, blood, fistula (such as anal fistula) and vessel such as blood vessel and/or lymph vessel.

Preferably, the method comprises the following steps:
a) inserting and positioning an endoscope into the inner body cavity of the human or non-human mammal by passing the endoscope through the first flexible tube of the medical device according to the first aspect of the present invention,
b) extending the distal end portion of the second flexible tube of the medical device according to the first aspect of the present invention inside the inner body cavity of the human or non-human mammal, while the inner body cavity is viewed by the endoscope, in particular by pushing a guidewire inwards and using markings or marks of the guidewire as reference,
c) inserting a fluid drainage device or a fluid collection element of a fluid drainage device or a sheath at least partially or sectionally surrounding or encasing a fluid drainage device or a fluid collection element of a fluid drainage device into the second flexible tube of the medical device according to the first aspect of the present invention,
d) pushing the sheath (together with the fluid drainage device or fluid collection element of the fluid drainage device surrounded or encased by the sheath) through the second flexible tube of the medical device according to the first aspect of the present invention to the inner body cavity viewed by the endoscope,
e) releasing the fluid drainage device or fluid collection element of the fluid drainage device from the sheath, after the sheath has been deployed from the second flexible tube of the medical device according to the first aspect of the present invention, i.e. has been placed into the inner body cavity,
f) retracting the medical device according to the first aspect of the present invention and the (opened) sheath from the body of the human or non-human mammal while maintaining the fluid drainage device in place, and
g) connecting the fluid drainage device, in particular the fluid communication element of the fluid drainage device, to a negative pressure or vacuum source.

Preferably, step c) is carried out by using a feeding element being adapted to insert the fluid drainage device or the fluid collection element of the fluid drainage device into the sheath.

Further, the fluid drainage device or the fluid communication element of the fluid drainage device may be inserted into a pushing element and in particular locked to a groove present in a handle of the pushing element.

Further, the fluid drainage device or the fluid communication element of the fluid drainage device may be inserted into the feeding element and pushed via the pushing element until the entire fluid drainage device or fluid collection element of the fluid drainage device passes a marking indicating a proximal start of a release element being adapted to release the fluid drainage device or the fluid collection element of the fluid drainage device from the sheath.

Further, the pushing element may be removed and the feeding element may be unlocked, in particular unscrewed, to release the sheath at least partially or sectionally surrounding or encasing the fluid drainage device or the fluid collection element of the fluid drainage device from the feeding element.

Further, the fluid communication element of the fluid drainage device may be inserted into a further pushing element being different from the above-mentioned pushing element, in particular being longer than the above-mentioned pushing element, until the further pushing element contacts the fluid collection element, in particular a base of the fluid collection element.

Preferably, the fluid communication element is longer than the further pushing element, and thus protrudes through a proximal end portion of the further pushing element. Expediently, a fixator, in particular in the form of a clamp, is used to fix the fluid communication element, in particular at a base of the further pushing element. Thus, a falling of the fluid collection element during handling can be advantageously avoided.

Further, the endoscope is preferably passed through the first flexible tube of the medical device until the distal end portion of the endoscope shows up. Preferably, with an adhesive tape or adhesive flap placed at the distal end portion of the second flexible tube, the second flexible tube, and thus the medical device, may be fixed to the endoscope, in particular below the distal end portion of the endoscope.

Further, a first connecting element of the first flexible tube may be tightened to fix the medical device to the endoscope, in particular to the proximal end portion of the endoscope.

Further, a guidewire may be passed through a working channel of the endoscope until it comes out from the distal end portion of the endoscope. Further, the guidewire may be connected to a connecting element being located at the distal end portion of the second flexible tube. The connecting element may be, for example, in the form of a screw or a clip mechanism.

Further, a manipulating element, in particular in the form of a thread, of the second flexible tube and/or the guidewire may be pulled to retract the distal end portion of the second flexible tube, and thus advantageously unblock a camera of the endoscope.

Further, the distal end portion of the second flexible tube is preferably deployed inside the inner body cavity by pushing the guidewire and using the markings or marks of the guidewire as a reference to know when the second flexible tube is totally extended, in particular until the entire fluid drainage device or fluid collection element of the fluid drainage device is released from the sheath.

Further, the endoscope may be retrieved outside the inner body cavity, in particular in such a way that the endoscope is preferably outside and the medical device according to the first aspect of the invention is inside the inner body cavity.

Further, the sheath may be pushed by means of the pushing element through the second flexible tube of the medical device until the sheath reaches the inner body cavity. Advantageously, the path of the sheath through the second flexible tube can be screened via the endoscope.

Once a marking of the above-mentioned further pushing element reaches an insertion guiding element of the second flexible tube, the sheath is outside the second flexible tube, and thus outside the medical device. Then the pulling element of the second flexible tube may be pulled to open the sheath, and thus to release the fluid drainage device or the fluid collection element of the fluid drainage device from the sheath. Further, the clamp may be removed and the other pushing element and the sheath may be removed from the patient by pulling a tail of the sheath. Further, the guidewire may be pulled to retract the medical device. Further, the fluid drainage device or the fluid collection element of the fluid drainage device may be checked with the endoscope and the endoscope may be removed.

Further, the fluid drainage device or the fluid collection element of the fluid drainage device may be connected, in particular via an extension tube, to a negative pressure or vacuum source, in particular to a negative pressure or vacuum pump or vacuum bottle.

Further, a negative pressure or vacuum may be applied, for example of 20 mmHg to 700 mmHg, in particular 30 mmHg to 300 mmHg, preferably 50 mmHg to 250 mmHg.

Further, the negative pressure or vacuum may be applied for 1 day to 31 days, in particular 1 day to 7 days, preferably 1 day to 5 days.

With respect to further features and advantages of the method, reference is made in its entirety to the previous description. The features and advantages disclosed therein do apply mutatis mutandis.

Further characteristics and advantages of the invention are provided in the following description of preferred embodiments in the form of figures and figure descriptions and the subclaims. These may concern individual embodiments of characteristics or embodiments combining several characteristics. The embodiments described serve only to explain the present invention, which is by no means limited to said embodiments and which is defined by the appended claims.

The figures schematically show the following:
- Fig. 1a-d:: an embodiment of a medical device according to the present disclosure,
- Fig. 2a-e:: an embodiment of a medical kit according to the present invention,
- Fig. 3-5:: embodiments of a sheath according to the present disclosure,

- Fig. 6:: an embodiment of a further medical kit according to the present invention and
- Figs. 7a-e: a further embodiment of a medical kit according to the present invention.

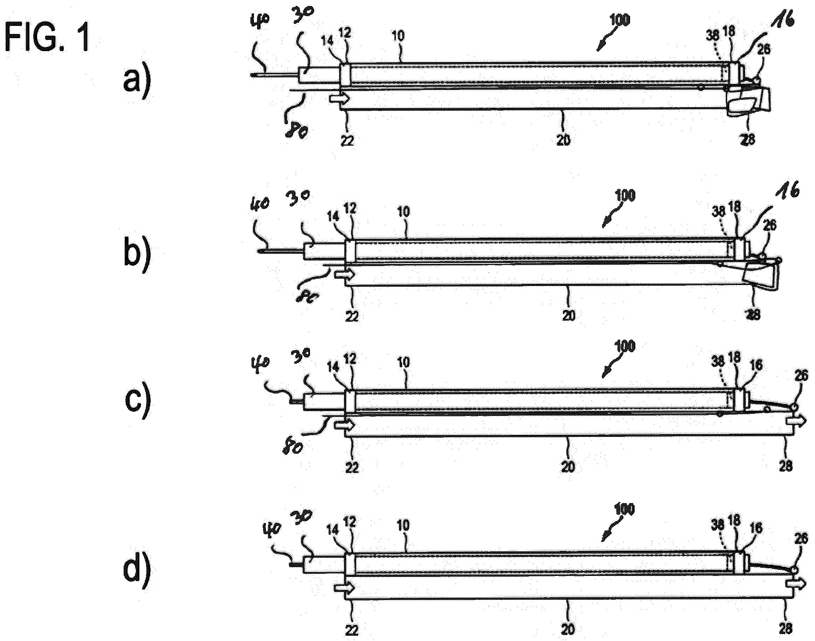

Fig. 1a-d schematically shows an embodiment of the medical device 100.

The medical device 100 comprises
- a first flexible, in particular compressible and/or pliant, tube 10 having a proximal end portion 12 and a distal end portion 18
   and
- a second flexible, in particular compressible and/or pliant, tube 20 having a proximal end portion 22 and a distal end portion 28.

The first flexible tube 10 is adapted to insert an endoscope 30 into a patient's body.

The second flexible tube 20 is adapted to insert a fluid drainage device, in particular a fluid drainage device being at least partially or sectionally, in particular only partially or sectionally or completely, surrounded by a sheath, into a patient's body, wherein the fluid drainage device is adapted to drain body fluids.

Preferably, the second flexible tube 20, in particular the distal end portion 28 of the second flexible tube 20, overhangs the distal end portion 18 of the first flexible tube 10.

Further, the first flexible tube 10 and the second flexible tube 20 are, in particular at least partially or sectionally, in particular only partially or sectionally or continuously, connected or joined together lengthwise, i.e. lengthways or longitudinal.

Further, the medical device 100 may have a first connecting element 14 and a second connecting element 16. The first connecting element 14 may be located at the proximal end portion 12 of the first flexible tube 10 and is adapted to connect, in particular positive connect and/or frictionally connect, a proximal end portion of the endoscope to the proximal end portion 12 of the first flexible tube 10. For example, the first connecting element 14 may have a hollow form, for example a hollow cylindrical form or hollow conical form. More preferably, the hollow form may have an adjustable inner diameter, wherein the inner diameter of the hollow form is preferably adjustable via a fixation element such as a rotary knob or screw.

The second connecting element 16 may be located at the distal end portion 28 of the second flexible tube 20 or at a portion of the second flexible tube 20 being located proximal to the distal end portion 28 of the flexible tube 20 and is adapted to connect, in particular materially bond, a distal end portion 38 of the endoscope 30 to the distal end portion 28 of the second flexible tube 20 or to the portion of the second flexible tube 20 being located proximal to the distal end portion 28 of the flexible tube 20.

Preferably, the second connecting element 16 is in the form of at least one adhesive tape or at least one adhesive flap.

Further, the medical device 100 may comprise a manipulating element 40. Preferably, the manipulating element 40 is connected to the distal end portion 28 of the second flexible tube 20. The manipulating element 40 is preferably adapted to retract, in particular fold back or roll up, or stretch, in particular unfold or unroll, the distal end portion 28 of the second flexible tube 20. Thus, a blocking of a camera of the endoscope 30 may be advantageously circumvented.

The manipulating element 40 may be preferably in the form of a longitudinal element, in particular in the form of a thread or wire.

Further, the medical device 100 preferably has a third connecting element 26 being located at the distal end portion 28 of the second flexible tube 20. Preferably, the connecting element 26 is adapted to connect a distal end portion 48 of the manipulating element 40 to the distal end portion 28 of the second flexible tube 20 and to disconnect the distal end portion 48 of the manipulating element 40 from the distal end portion 28 of the second flexible tube 20. The connecting element 26 may be, for example, in the form of a screw being fixed, in particular materially bonded, for example welded, to the distal end portion 28 of the second flexible tube 20. Thus, depending on the movement of the manipulating element 40, the distal end portion 28 of the second flexible tube 20 may be either retracted, in particular folded or compressed, or stretched, in particular unfolded or expanded. Thus, the intracorporal cavity to be treated can be viewed via the endoscope 30 without any impairments.

Further, the medical device 100 preferably comprises a pulling element 80. Preferably, the pulling element 80 is adapted to fold the distal end portion 28 of the second flexible tube 20, in particular in the direction or towards the proximal end portion 22 of the second flexible tube 20. Thus, avoidance of blocking of a camera of the endoscope 30 may be advantageously further optimized.

Preferably, the pulling element 80 is attached or connected, in particular sewn, stitched or knotted, to the distal end portion 28, in particular to a middle of the distal end portion 28 or along a longitudinal side of the distal end portion 28, of the second flexible tube 20, in particular by means of at least one knot, i.e. only one knot or a plurality of knots, for example two or more knots.

Further, the pulling element 80 may be arranged or located at least sectionally, in particular only sectionally, inside the second flexible tube 20 and in particular up to the proximal end portion 22 of the second flexible tube 20.

Further, the pulling element 80 may be in the form of a wire, in particular plastic wire or metal wire.

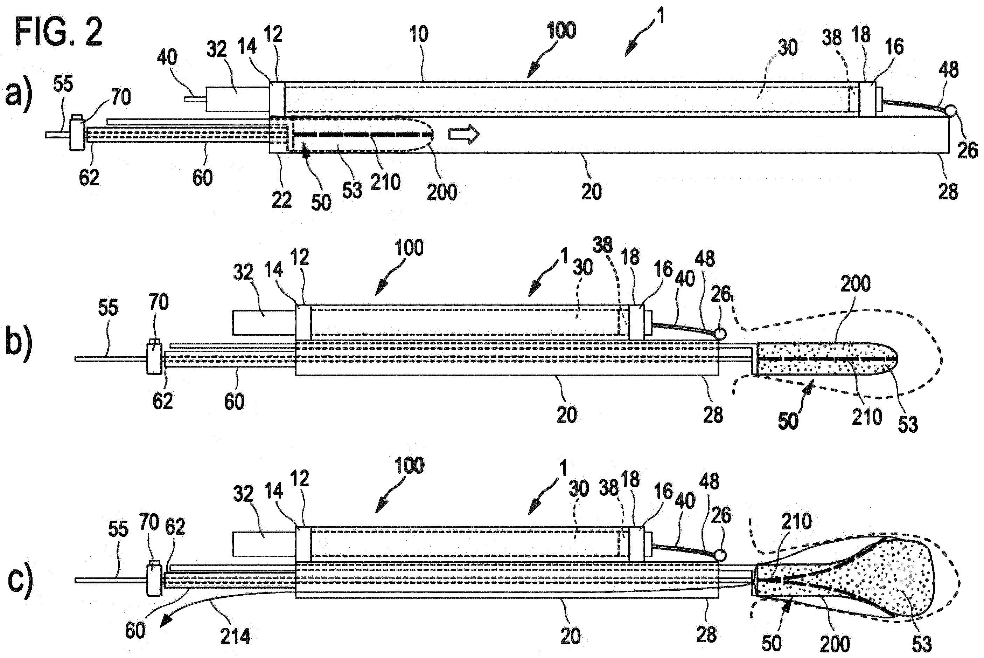

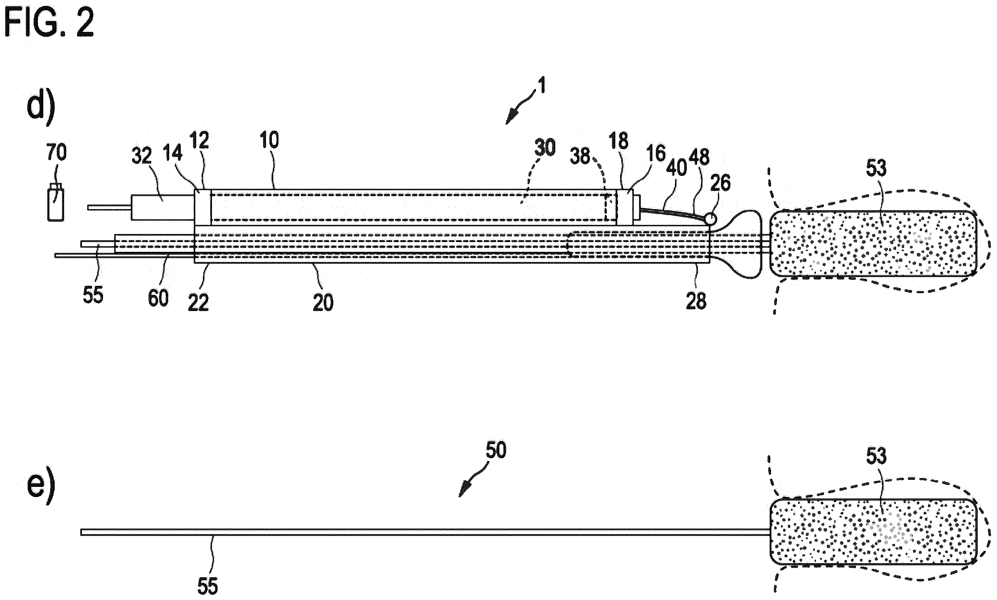

Fig. 2a-e schematically shows an embodiment of a medical kit 1 according to the present invention.

The medical kit 1 is in particular for treating an intracorporal cavity such as colon or esophagus of a human or non-human mammal and/or for carrying out an intracorporal, preferably endoluminal and/or endocavitary, vacuum therapy.

The medical kit 1 comprises, in particular spatially separated from each other, at least the following components:
- a medical device 100 and
- a fluid drainage device 50 being adapted to drain body fluids and
- optionally a sheath 200 being adapted to surround or encase the fluid drainage device 50 at least partially or sectionally, in particular only partially or sectionally or completely, or a sheath 200 surrounding or encasing the fluid drainage device 50 at least partially or sectionally, in particular only partially or sectionally or completely.

The medical device 100 comprises
- a first flexible, in particular compressible and/or pliant, tube 10 having a proximal end portion 12 and a distal end portion 18
   and
- a second flexible, in particular compressible and/or pliant, tube 20 having a proximal end portion 22 and a distal end portion 28.

The first flexible tube 10 is adapted to insert an endoscope 30 into a patient's body.

The second flexible tube 20 is adapted to insert the fluid drainage device 50, in particular being at least partially or sectionally, in particular only partially or sectionally or completely, surrounded by the sheath 200, into a patient's body.

Preferably, the second flexible tube 20, in particular the distal end portion 28 of the second flexible tube 20, overhangs the distal end portion 18 of the first flexible tube 10.

Further, the first flexible tube 10 and the second flexible tube 20 are, in particular at least partially or sectionally, in particular only partially or sectionally or continuously, connected or joined together lengthwise, i.e. lengthways or longitudinal.

The fluid drainage device 50 comprises a fluid collection element 53 and a fluid communication element 55. Preferably, the fluid collection element 53 has a foamy or spongy structure. More preferably, the fluid collection element 53 is in the form of a foam or sponge structure, in particular an open-pored foam or sponge structure, which, for example, comprises or consists of polyurethane. The fluid communication element 55 is preferably in the form of a drainage tube. Preferably, a distal end portion of the fluid communication element 55 is surrounded or encased by the fluid collection element 53.

The distal end portion of the fluid communication element 55 preferably has holes or perforations breaking through the wall of the fluid communication element 55. Thus, a fluidic connection between the fluid collecting element 53 and the fluid communication element 55 is achieved. The fluid collection element 53 is adapted to collect, in particular absorb or soak, body fluids, in particular pathological body fluids.

Further, the second flexible tube 20 may have an inner diameter being smaller than a maximum diameter of the fluid collection element 53, in particular being surrounded or encased by the sheath 200. Thus, the fluid collection element 53, in particular being surrounded or encased by the sheath 200, may be compressed when inserted into and pulled through the second flexible tube 20.

Further, the medical device 100 may have a first connecting element 14 and a second connecting element 16. The first connecting element 14 may be located at the proximal end portion 12 of the first flexible tube 10 and is adapted to connect, in particular positive connect and/or frictionally connect, a proximal end portion of the endoscope to the proximal end portion 12 of the first flexible tube 10. For example, the first connecting element 14 may have a hollow form, for example a hollow cylindrical form or hollow conical form. More preferably, the hollow form may have an adjustable inner diameter, wherein the inner diameter of the hollow form is preferably adjustable via a fixation element such as a rotary knob or screw.

The second connecting element 16 may be located at the distal end portion 28 of the second flexible tube 20 or at a portion of the second flexible tube 20 being located proximal to the distal end portion 28 of the flexible tube 20 and is adapted to connect, in particular materially bond, a distal end portion 38 of the endoscope 30 to the distal end portion 28 of the second flexible tube 20 or to the portion of the second flexible tube 20 being located proximal to the distal end portion 28 of the flexible tube 20.

Preferably, the second connecting element 16 is in the form of at least one adhesive tape or at least one adhesive flap.

Further, the medical device 100 may comprise a manipulating element 40. Preferably, the manipulating element 40 is connected to the distal end portion 28 of the second flexible tube 20. The manipulating element 40 is preferably adapted to retract, in particular fold back or roll up, or stretch, in particular unfold or unroll, the distal end portion 28 of the second flexible tube 20. Thus, a blocking of a camera of the endoscope 30 may be advantageously circumvented.

The manipulating element 40 may be preferably in the form of a longitudinal element, in particular in the form of a thread or wire.

Further, the medical device 100 preferably has a third connecting element 26 being located at the distal end portion 28 of the second flexible tube 20. Preferably, the connecting element 26 is adapted to connect a distal end portion 48 of the manipulating element 40 to the distal end portion 28 of the second flexible tube 20 and to disconnect the distal end portion 48 of the manipulating element 40 from the distal end portion 28 of the second flexible tube 20. The connecting element 26 may be, for example, in the form of a screw being fixed, in particular materially bonded, for example welded, to the distal end portion 28 of the second flexible tube 20. Thus, depending on the movement of the manipulating element 40, the distal end portion 28 of the second flexible tube 20 may be either retracted, in particular folded or compressed, or stretched, in particular unfolded or expanded. Thus, the intracorporal cavity to be treated can be viewed via the endoscope 30 without any impairments.

Further, the medical kit 1 may comprise an insertion guiding element 60. The insertion guiding element 60 is adapted to push the fluid drainage device 50, in particular with the fluid collection element 53 being surrounded or encased by the sheath 200, through the second flexible tube 20. To avoid uncontrolled movement of the fluid drainage device 50, in particular of the fluid collection element 53, during the treatment, the medical kit may further comprise a locking mechanism, in particular a clamp, 70. Thus, the fluid communicating element 55 may be fixed, in particular close to the proximal end portion 62 of the insertion guiding element 60. Thus, stability of the fluid drainage device 50 may be advantageously maintained during insertion.

If the sheath 200 with the fluid collection element 53 inside the sheath 200 has reached the intracorporal cavity to be treated, the fluid collection element 53 is preferably released from the sheath 200. To this means, the sheath 200 has in particular a release element 210, in particular in the form of an openable closure element, preferably in the form of a zip mechanism. More preferably, the release element 210 comprises a longitudinal element 214, in particular in the form of a thread or wire, being stitched to the sheath 200.

After release of the fluid collection element 53 from the sheath 200, the locking mechanism 70 and the insertion guiding element 60 are preferably removed.

Further, the sheath 200 preferably has a tail. Thus, the sheath 200 may be conveniently removed from the patient's body after release of the fluid collection element 53. Further, after release of the fluid collection element 53, the guidewire 40 is preferably pulled to retract the second flexible tube 20, in particular a distal end portion of the second flexible tube 20.

Further, the fluid collection element 53 may be checked with the endoscope 30 and subsequently the endoscope 30 may be removed.

Finally, the fluid communication element 55 can be connected, in particular via an extension tube, to a negative pressure or vacuum source, in particular to a negative pressure or vacuum pump.

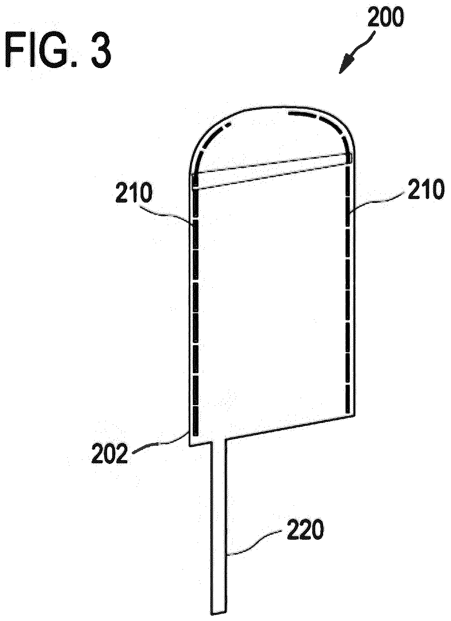

Fig. 3 schematically shows an embodiment of a sheath 200 according to the present invention.

The sheath 200 is adapted to surround or encase a fluid drainage device at least partially or sectionally, in particular only partially or sectionally or completely.

Preferably, the sheath 200 has at least partially or sectionally, in particular only partially or sectionally or continuously, a tubular form.

More preferably, the sheath 200 is in the form of a capsule or has a capsule form. Further preferably, the sheath 200 is flexible, in particular compressible or pliant.

Further, the sheath 200 preferably has a release element 210 being adapted to release a fluid drainage device from the sheath 200.

More specifically, the release element 210 may be in the form of an openable closure element, preferably a zip mechanism.

Further, the release element 210 preferably extends along at least one longitudinal side, in particular along two oppositely arranged longitudinal sides, of the sheath 200.

More specifically, the release element 210 may comprise a perforation or material thinning or may be in the form of a perforation or material thinning. Alternatively or in combination, the release element 210 may comprise a longitudinal element, in particular thread or wire, being connected, in particular sewn, i.e. stitched, to the sheath 200, in particular to a distal end portion of the sheath 200. Thus, the fluid collection element 53 of the fluid drainage device 50 may be advantageously released from the sheath 200 by pulling the longitudinal element, and thus undoing the sewing or stitching of the longitudinal element to the sheath 200.

Further, the sheath 200 may comprise a tail, in particular longitudinal tail, 220. Preferably, the tail 220 is arranged at a proximal end portion 202 of the sheath 200. The tail 220 is adapted to retract the sheath 200 after release of the fluid drainage device from the sheath 200.

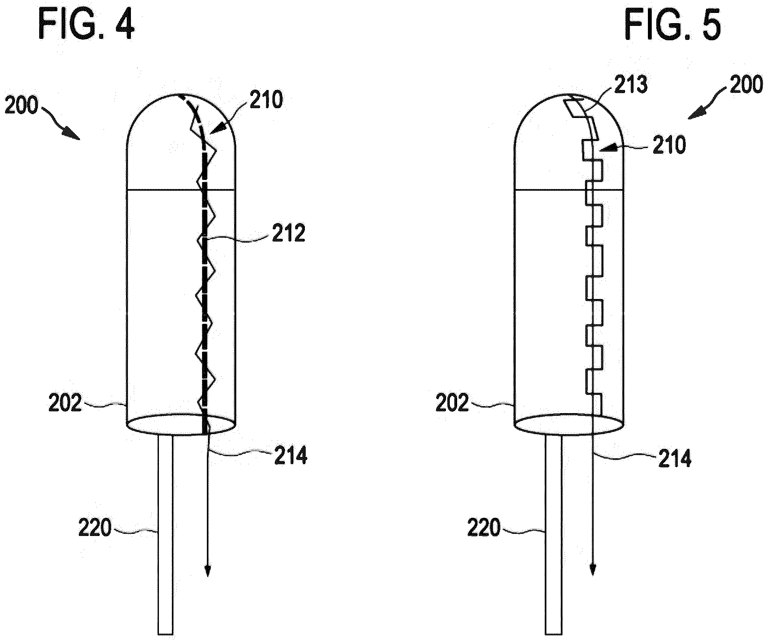

Fig. 4 shows a further embodiment of a sheath 200 according to the present invention.

The sheath 200 is adapted to surround or encase a fluid drainage device at least partially or sectionally, in particular only partially or sectionally or completely.

The sheath 200 has a release element 210 being adapted to release the fluid drainage device from the sheath 200. The release element 210 comprises a perforation 212 and a longitudinal element 214, in particular in the form of a thread or wire, being connected, in particular sewn, i.e. stitched, to the sheath 200. Thus, the fluid drainage device may be advantageously released from the sheath 200 by pulling the longitudinal element 214, and thus undoing the sewing or stitching of the longitudinal element 214 to the sheath 200 and concurrently tearing the perforation 212.

Further, the sheath 200 may comprise a tail, in particular longitudinal tail, 220. Preferably, the tail 220 is arranged at a proximal end portion 202 of the sheath 200. The tail 220 is adapted to retract the sheath 200 after release of the fluid drainage device from the sheath 200.

Fig. 5 shows a further embodiment of a sheath 200 according to the present invention.

The sheath 200 is adapted to surround or encase a fluid drainage device at least partially or sectionally, in particular only partially or sectionally or completely.

The sheath 200 has a release element 210 being adapted to release the fluid drainage device from the sheath 200. The release element 210 comprises a material thinning 213 and a longitudinal element 214, in particular in the form of a thread or wire, being connected, in particular sewn, i.e. stitched, to the sheath 200. Thus, the fluid drainage device may be advantageously released from the sheath 200 by pulling the longitudinal element 214, and thus undoing the sewing or stitching of the longitudinal element 214 to the sheath 200 and concurrently tearing the material thinning 213.

Further, the sheath 200 may comprise a tail, in particular longitudinal tail, 220. Preferably, the tail 220 is arranged at a proximal end portion 202 of the sheath 200. The tail 220 is adapted to retract the sheath 200 after release of the fluid drainage device from the sheath 200.

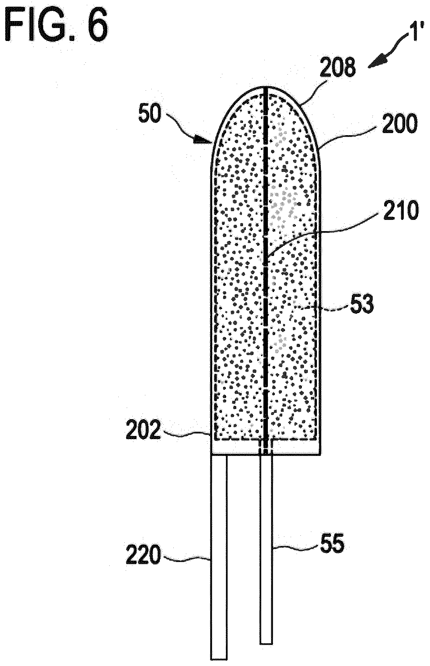

Fig. 6 schematically displays an embodiment of a further medical kit 1' according to the present invention.

The medical kit 1' comprises a fluid drainage device 50 and a sheath 200.

The fluid drainage device 50 and the sheath 200 may be spatially separated from each other or the fluid drainage device 50 may be at least partially or sectionally, in particular only partially or sectionally, surrounded or encased by the sheath 200.

The fluid drainage device 50 preferably comprises a fluid collection element 53 and a fluid communication element 55. The fluid collection element 53 is expediently adapted for collect, in particular absorb or soak, body fluids, in particular pathological body fluids. More preferably, the fluid collection element 53 may have a foamy or spongy structure or may be in the form of a foam or sponge, in particular open-pored foam or sponge. The fluid communication element 55 is preferably in the form of a fluid drainage tube. Expediently, the fluid communication element 55 is attached to the fluid collection element 53. More specifically, a distal end portion of the fluid communication element 55 is preferably surrounded or encased by the fluid collection element 53.

Preferably, the sheath 200 has, in particular at least partially or sectionally, a tubular shape. More preferably, the sheath 200 is in the form of a capsule, wherein a proximal end portion 202 of the sheath 200 is open and a distal end portion 208 of the sheath 200 is closed.

Preferably, the sheath 200 is in the form of a flexible, in particular compressible or pliant sheath.

Further, the sheath 200, in particular in an expanded or unfolded state, has an inner diameter being smaller than an outer diameter, in particular maximum outer diameter, of the fluid collection element 53. Thus, the fluid collection element 53 may be compressed during its insertion into the sheath 200.

Further, the sheath 200 has a release element 210, in particular in the form of an openable closure element, preferably a zip mechanism. Further, the release element 210 preferably extends along at least one longitudinal side, in particular along two oppositely arranged longitudinal sides, of the sheath 200. For example, the release element 210 may be in the form of a perforation or material thinning. Alternatively or in combination, the release element 210 may comprise a pulling element, in particular in the form of a thread or wire, being sewn or stitched to the sheath 200 and also extending along at least one longitudinal side, in particular along two oppositely arranged longitudinal sides, of the sheath 200.

Further, the sheath 200 may have a tail 220. The tail 220 is advantageously adapted to retract or remove the sheath 200 from a patient's body after release of the fluid collection element 53 from the sheath 200.

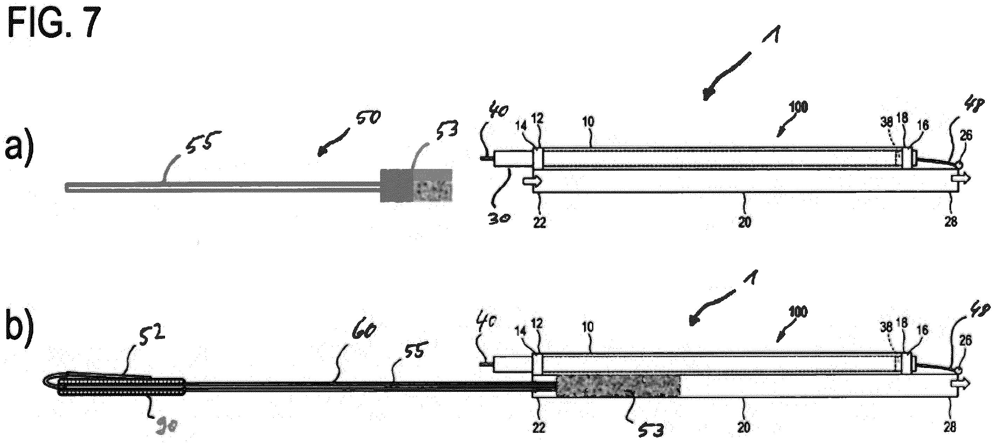

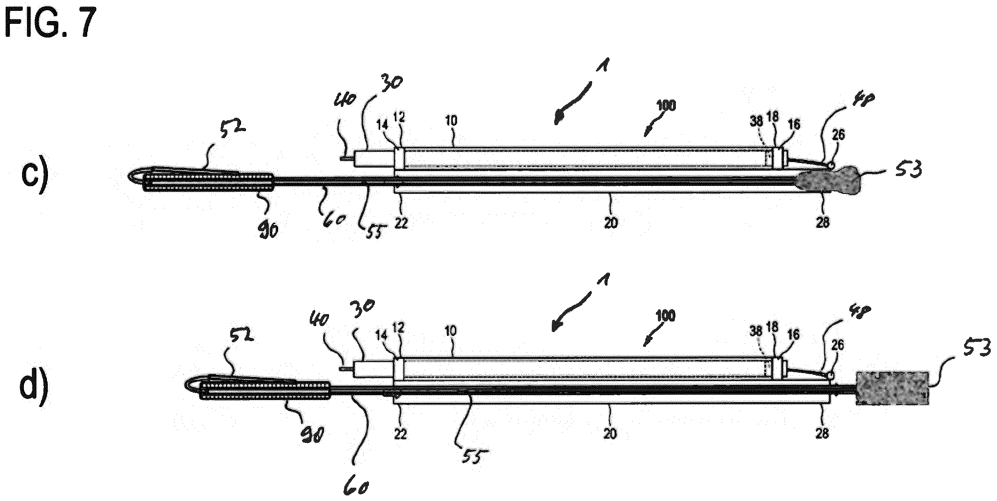

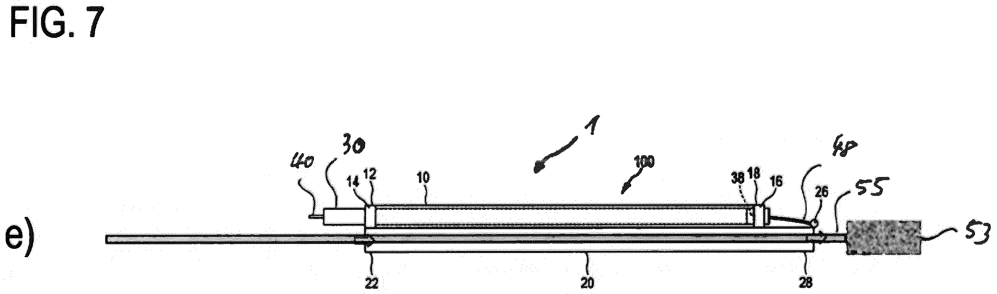

Figs. 7a-e schematically display a further embodiment of a medical kit 1 according to the present invention.

The medical kit 1 is in particular for treating an intracorporal cavity such as colon or esophagus of a human or non-human mammal and/or for carrying out an intracorporal, preferably endoluminal and/or endocavitary, vacuum therapy.

The medical kit 1 comprises, in particular spatially separated from each other, at least the following components:
- a medical device 100 and
- a fluid drainage device 50 being adapted to drain body fluids.

The medical device 100 comprises
- a first flexible, in particular compressible and/or pliant, tube 10 having a proximal end portion 12 and a distal end portion 18
   and
- a second flexible, in particular compressible and/or pliant, tube 20 having a proximal end portion 22 and a distal end portion 28.

The first flexible tube 10 is adapted to insert an endoscope 30 into a patient's body.

The second flexible tube 20 is adapted to insert the fluid drainage device 50 into a patient's body.

Preferably, the second flexible tube 20, in particular the distal end portion 28 of the second flexible tube 20, overhangs the distal end portion 18 of the first flexible tube 10.

Further, the first flexible tube 10 and the second flexible tube 20 are, in particular at least partially or sectionally, in particular only partially or sectionally or continuously, connected or joined together lengthwise, i.e. lengthways or longitudinal.

The fluid drainage device 50 comprises a fluid collection element 53 and a fluid communication element 55. Preferably, the fluid collection element 53 has a foamy or spongy structure. More preferably, the fluid collection element 53 is in the form of a foam or sponge structure, in particular an open-pored foam or sponge structure, which, for example, comprises or consists of polyurethane. The fluid communication element 55 is preferably in the form of a drainage tube. Preferably, a distal end portion of the fluid communication element 55 is surrounded or encased by the fluid collection element 53.

The distal end portion of the fluid communication element 55 preferably has holes or perforations breaking through the wall of the fluid communication element 55. Thus, a fluidic connection between the fluid collecting element 53 and the fluid communication element 55 is achieved. The fluid collection element 53 is adapted to collect, in particular absorb or soak, body fluids, in particular pathological body fluids.

Further, the second flexible tube 20 may have an inner diameter being smaller than a maximum diameter of the fluid collection element 53. Thus, the fluid collection element 53 may be compressed when inserted into and pulled through the second flexible tube 20.

Further, the medical device 100 may have a first connecting element 14 and a second connecting element 16. The first connecting element 14 may be located at the proximal end portion 12 of the first flexible tube 10 and is adapted to connect, in particular positive connect and/or frictionally connect, a proximal end portion of the endoscope to the proximal end portion 12 of the first flexible tube 10. For example, the first connecting element 14 may have a hollow form, for example a hollow cylindrical form or hollow conical form. More preferably, the hollow form may have an adjustable inner diameter, wherein the inner diameter of the hollow form is preferably adjustable via a fixation element such as a rotary knob or screw.

The second connecting element 16 may be located at the distal end portion 28 of the second flexible tube 20 or at a portion of the second flexible tube 20 being located proximal to the distal end portion 28 of the flexible tube 20 and is adapted to connect, in particular materially bond, a distal end portion 38 of the endoscope 30 to the distal end portion 28 of the second flexible tube 20 or to the portion of the second flexible tube 20 being located proximal to the distal end portion 28 of the flexible tube 20.

Preferably, the second connecting element 16 is in the form of at least one adhesive tape or at least one adhesive flap.

Further, the medical device 100 may comprise a manipulating element 40. Preferably, the manipulating element 40 is connected to the distal end portion 28 of the second flexible tube 20. The manipulating element 40 is preferably adapted to retract, in particular fold back or roll up, or stretch, in particular unfold or unroll, the distal end portion 28 of the second flexible tube 20. Thus, a blocking of a camera of the endoscope 30 may be advantageously circumvented.

The manipulating element 40 may be preferably in the form of a longitudinal element, in particular in the form of a thread or wire.

Further, the medical device 100 preferably has a third connecting element 26 being located at the distal end portion 28 of the second flexible tube 20. Preferably, the connecting element 26 is adapted to connect a distal end portion 48 of the manipulating element 40 to the distal end portion 28 of the second flexible tube 20 and to disconnect the distal end portion 48 of the manipulating element 40 from the distal end portion 28 of the second flexible tube 20. The connecting element 26 may be, for example, in the form of a screw being fixed, in particular materially bonded, for example welded, to the distal end portion 28 of the second flexible tube 20. Thus, depending on the movement of the manipulating element 40, the distal end portion 28 of the second flexible tube 20 may be either retracted, in particular folded or compressed, or stretched, in particular unfolded or expanded. Thus, the intracorporal cavity to be treated can be viewed via the endoscope 30 without any impairments.

Further, the medical kit 1 may comprise an insertion guiding element 60. The insertion guiding element 60 is adapted to push or insert the fluid drainage device 50 into and/or through the second flexible tube 20.

During insertion, a proximal end portion 52 of the fluid drainage device 50, preferably a proximal end portion 52 of the fluid communication element 55 of the fluid drainage device 50, is preferably connected, in particular positively connected and/or frictionally connected and/or materially bonded, to a handle 90 of the medical device 100. For example, the proximal end portion 52 of the fluid drainage device 50 may be trapped in grooves or recesses of the handle 90. Thus, stability of the fluid drainage device 50 may be advantageously maintained during insertion. The handle 90 may be part of the insertion guiding element 60. In particular, the handle 90 and the insertion guiding element 60 may be in the form of a one-piece construction. Further, the handle 90 may be attached to the insertion guiding element 60 or may be pushed onto the insertion guiding element 60. Alternatively, the handle 90 may be in the form of a separate element of the medical device 100, in particular being independent from the insertion guiding element 60. Further, the handle 90 may be arranged or located at or onto a proximal end portion of the insertion guiding element 60.

After deploying the fluid collection element 53 into the intracorporal cavity to be treated, the handle 90 and the insertion guiding element 60 are preferably removed.

Further, the fluid collection element 53 may be checked with the endoscope 30 and subsequently the endoscope 30 may be removed.

Finally, the fluid communication element 55 can be connected, in particular via an extension tube, to a negative pressure or vacuum source, in particular to a negative pressure or vacuum pump.

With respect to further features and advantages of the embodiments shown in the Figures, reference is made in its entirety to the general description.

## Claims

1. A medical kit, in particular for carrying out an intracorporal, preferably endoluminal and/or endocavitary, vacuum therapy, comprising
- a fluid drainage device (50) being adapted to drain body fluids and being at least partially or sectionally surrounded by a sheath (200),
- a medical device (100) comprising
a first flexible tube (10) having a proximal end portion (12) and a distal end portion (18) and
a second flexible tube (20) having a proximal end portion (22) and a distal end portion (28),
wherein the first flexible tube (10) is adapted to insert an endoscope (30) into a patient's body and the second flexible tube (20) is adapted to insert the fluid drainage device (50) into a patient's body, and wherein the first flexible tube (10) and the second flexible tube (20) are connected to each other lengthwise.

2. The medical kit according to claim 1, wherein the medical device (100) is **characterized in that** the second flexible tube (20), in particular the distal end portion (28) of the second flexible tube (20), overhangs the distal end portion (18) of the first flexible tube (10).

3. The medical kit according to claim 1 or 2, **characterized in that** the medical device (100) further comprises a first connecting element (14) and a second connecting element (16), wherein the first connecting element (14) is adapted to connect, in particular positive connect and/or frictionally connect, a proximal end portion of the endoscope (30) to the proximal end portion (12) of the first flexible tube (10) and the second connecting element (16) is adapted to connect, in particular materially bond, a distal end portion (38) of the endoscope (30) to the distal end portion (28) of the second flexible tube (20) or to a portion of the second flexible tube (20) being located proximal to the distal end portion (28) of the second flexible tube (20).

4. The medical kit according to any of the preceding claims, **characterized in that** the medical device (100) further comprises a manipulating element (40) being adapted to retract or stretch the distal end portion (28) of the second flexible tube (20).

5. The medical kit according to claim 4, wherein the medical device (100) is **characterized in that** the manipulating element (40) is in the form of a longitudinal element (214), in particular thread or wire, being attached, in particular sewn or knotted, to the distal end portion (28) of the second flexible tube (20).

6. The medical kit according to claim 4 or 5, wherein the medical device (100) is **characterized in that** a distal end portion (48) of the manipulating element (40) has at least one mark.

7. The medical kit according to any of the claims 4 to 6, **characterized in that** the medical device (100) further comprises a third connecting element (26) being adapted to connect a distal end portion (48) of the manipulating element (40) to the distal end portion (28) of the second flexible tube (20) and to disconnect the distal end portion (48) of the manipulating element (40) from the distal end portion (28) of the second flexible tube (20).

8. The medical kit according to any of the preceding claims, **characterized in that** the medical device (100) further comprises a pulling element (80), in particular in the form of a wire, being adapted to fold the distal end portion (28) of the second flexible tube (20) in the direction of the proximal end portion (22) of the second flexible tube (20).

9. The medical kit according to any of the preceding claims, **characterized in that** the medical device (100) further has an insertion guiding element (60) being located at the proximal end portion (22) of the second flexible tube (20) and being adapted to push the fluid drainage device (50), in particular fluid drainage device (50) being at least partially or sectionally surrounded by a sheath (200), into the second flexible tube (20).

10. The medical kit according to any of the preceding claims, **characterized in that** a handle (90) of the medical device (100) or the insertion guiding element (60) is adapted to be connected or is connected to the fluid drainage device (50), in particular to a proximal end portion (52) of the fluid drainage device (50).

11. The medical kit according to any of the claims 3 to 10, wherein the medical device (100) is **characterized in that** the first connecting element (14) and the insertion guiding element (60) are formed together in one piece, wherein preferably a distal end portion of the first connecting element (14) is inserted into/inside the proximal end portion (12) of the first flexible tube (10) and a distal end portion of the insertion guiding element (60) is inserted into/inside the proximal end portion (22) of the second flexible tube (20).

12. Medical kit according to any of the preceding claims, **characterized in that** the fluid drainage device (50) comprises a fluid collection element (53), in particular having a foamy or spongy structure, and a fluid communication element (55).

13. Medical kit according to any of the preceding claims, **characterized in that** the sheath (200) has a release element (210) being adapted to release the fluid drainage device (50) or the fluid collection element (53) from the sheath (200).

14. Medical kit according to any of the preceding claims, **characterized in that** a longitudinal tail (220) is formed at a proximal end portion (202) of the sheath (200) and/or the sheath (200) has a marking indicating a proximal start of the release element (210).

15. Medical kit according to any of the preceding claims, **characterized in that** the medical kit comprises at least one further kit component being selected from the group consisting of feeding element, manipulating element, pulling element, at least one pushing element, clamp, endoscope, syringe, dilatator extensor tube, tube connectors such as luer lock connector and/or Y-connector, connector being adapted to be arranged between the fluid communication element and a/the dilatator extensor tube, lubricant, wipes, scissors, instructions for use and combinations of at least two of the afore-said further kit components.

## Patentansprüche

1. Medizinisches Set, insbesondere zur Durchführung einer intrakorporalen, bevorzugt endoluminalen und/oder endokavitären, Vakuumtherapie, aufweisend
- eine Flüssigkeitsdrainagevorrichtung (50), die zum Ableiten von Körperflüssigkeiten geeignet ist und mindestens teilweise oder abschnittsweise von einer Hülle (200) umgeben ist,
- eine medizinische Vorrichtung (100), aufweisend
einen ersten flexiblen Schlauch (10) mit einem proximalen Endabschnitt (12) und einem distalen Endabschnitt (18) und
einen zweiten flexiblen Schlauch (20) mit einem proximalen Endabschnitt (22) und einem distalen Endabschnitt (28),
wobei der erste flexible Schlauch (10) zum Einführen eines Endoskops (30) in den Körper eines Patienten geeignet ist und der zweite flexible Schlauch (20) zum Einführen der Flüssigkeitsdrainagevorrichtung (50) in den Körper eines Patienten geeignet ist, und wobei der erste flexible Schlauch (10) und der zweite flexible Schlauch (20) in Längsrichtung miteinander verbunden sind.

2. Medizinisches Set nach Anspruch 1, wobei die medizinische Vorrichtung (100) **dadurch gekennzeichnet ist, dass** der zweite flexible Schlauch (20), insbesondere der distale Endabschnitt (28) des zweiten flexiblen Schlauchs (20), den distalen Endabschnitt (18) des ersten flexiblen Schlauchs (10) überragt.

3. Medizinisches Set nach Anspruch 1 oder 2, **dadurch gekennzeichnet, dass** die medizinische Vorrichtung (100) ferner ein erstes Verbindungselement (14) und ein zweites Verbindungselement (16) aufweist, wobei das erste Verbindungselement (14) zum Verbinden, insbesondere zum formschlüssigen und/oder kraftschlüssigen Verbinden, eines proximalen Endabschnitts des Endoskops (30) mit dem proximalen Endabschnitt (12) des ersten flexiblen Schlauchs (10) geeignet ist und das zweite Verbindungselement (16) zum Verbinden, insbesondere zum stoffschlüssigen Verbinden, eines distalen Endabschnitts (38) des Endoskops (30) mit dem distalen Endabschnitt (28) des zweiten flexiblen Schlauchs (20) oder mit einem Abschnitt des zweiten flexiblen Schlauchs (20), der sich proximal zum distalen Endabschnitt (28) des zweiten flexiblen Schlauchs (20) befindet, geeignet ist.

4. Medizinisches Set nach einem der vorstehenden Ansprüche, **dadurch gekennzeichnet, dass** die medizinische Vorrichtung (100) ferner ein Manipulationselement (40) aufweist, das zum Zurückziehen oder zum Strecken des distalen Endabschnitts (28) des zweiten flexiblen Schlauchs (20) geeignet ist.

5. Medizinisches Set nach Anspruch 4, wobei die medizinische Vorrichtung (100) **dadurch gekennzeichnet ist, dass** das Manipulationselement (40) die Form eines länglichen Elements (214), insbesondere eines Fadens oder Drahts, aufweist, der am distalen Endabschnitt (28) des zweiten flexiblen Schlauchs (20) angebracht, insbesondere daran angenäht oder verknotet ist.

6. Medizinisches Set nach Anspruch 4 oder 5, wobei die medizinische Vorrichtung (100) **dadurch gekennzeichnet ist, dass** ein distaler Endabschnitt (48) des Manipulationselements (40) mindestens eine Markierung aufweist.

7. Medizinisches Set nach einem der Ansprüche 4 bis 6, **dadurch gekennzeichnet, dass** die medizinische Vorrichtung (100) ferner ein drittes Verbindungselement (26) aufweist, das zum Verbinden eines distalen Endabschnitts (48) des Manipulationselements (40) mit dem distalen Endabschnitt (28) des zweiten flexiblen Schlauchs (20) und zum Trennen des distalen Endabschnitts (48) des Manipulationselements (40) von dem distalen Endabschnitt (28) des zweiten flexiblen Schlauchs (20) geeignet ist.

8. Medizinisches Set nach einem der vorstehenden Ansprüche, **dadurch gekennzeichnet, dass** die medizinische Vorrichtung (100) ferner ein Zugelement (80), insbesondere in Form eines Drahts, aufweist, das zum Klappen des distalen Endabschnitts (28) des zweiten flexiblen Schlauchs (20) in Richtung des proximalen Endabschnitts (22) des zweiten flexiblen Schlauchs (20) geeignet ist.

9. Medizinisches Set nach einem der vorstehenden Ansprüche, **dadurch gekennzeichnet, dass** die medizinische Vorrichtung (100) ferner ein Einführ-Führungselement (60) aufweist, das sich am proximalen Endabschnitt (22) des zweiten flexiblen Schlauchs (20) befindet und zum Schieben der Flüssigkeitsdrainagevorrichtung (50), insbesondere der Flüssigkeitsdrainagevorrichtung (50), die mindestens teilweise oder abschnittsweise von einer Hülle (200) umgeben ist, in den zweiten flexiblen Schlauch (20) geeignet ist.

10. Medizinisches Set nach einem der vorstehenden Ansprüche, **dadurch gekennzeichnet, dass** ein Handgriff (90) der medizinischen Vorrichtung (100) oder des Einführ-Führungselements (60) zum Verbinden mit der Flüssigkeitsdrainagevorrichtung (50), insbesondere mit einem proximalen Endabschnitt (52) der Flüssigkeitsdrainagevorrichtung (50) geeignet ist oder damit verbunden ist.

11. Medizinisches Set nach einem der Ansprüche 3 bis 10, wobei die medizinische Vorrichtung (100) **dadurch gekennzeichnet ist, dass** das erste Verbindungselement (14) und das Einführ-Führungselement (60) gemeinsam in einem Stück ausgebildet sind, wobei bevorzugt ein distaler Endabschnitt des ersten Verbindungselements (14) in den/innerhalb des proximalen Endabschnitts (12) des ersten flexiblen Schlauchs (10) und ein distaler Endabschnitt des Einführ-Führungselements (60) in den/innerhalb des proximalen Endabschnitts (22) des zweiten flexiblen Schlauchs (20) eingeführt ist.

12. Medizinisches Set nach einem der vorstehenden Ansprüche, **dadurch gekennzeichnet, dass** die Flüssigkeitsdrainagevorrichtung (50) ein Flüssigkeitssammelelement (53), insbesondere eines, das eine Schaum- oder Schwammstruktur aufweist, und ein Flüssigkeitsverbindungselement (55) aufweist.

13. Medizinisches Set nach einem der vorstehenden Ansprüche, **dadurch gekennzeichnet, dass** die Hülle (200) ein Löseelement (210) aufweist, das zum Lösen der Flüssigkeitsdrainagevorrichtung (50) oder des Flüssigkeitssammelelements (53) von der Hülle (200) geeignet ist.

14. Medizinisches Set nach einem der vorstehenden Ansprüche, **dadurch gekennzeichnet, dass** an einem proximalen Endabschnitt (202) der Hülle (200) ein längliches Endstück (220) ausgebildet ist und/oder die Hülle (200) eine Markierung aufweist, die einen proximalen Beginn des Löseelements (210) angibt.

15. Medizinisches Set nach einem der vorstehenden Ansprüche, **dadurch gekennzeichnet, dass** das medizinische Set mindestens eine weitere Set-Komponente aufweist, die aus der Gruppe bestehend aus einem Zuführelement, einem Manipulationselement, einem Zugelement, mindestens einem Schiebeelement, einer Klemme, einem Endoskop, einer Spritze, einem Dilatator-Dehnungsschlauch, Schlauchverbindern wie einem Luer-Lock-Verbinder und/oder einem Y-Verbinder, einem Verbinder, der dafür geeignet ist, zwischen dem Flüssigkeitsverbindungselement und einem/dem Dilatator-Dehnungsschlauch angeordnet zu werden, einem Gleitmittel, Tüchern, einer Schere, einer Gebrauchsanweisung und Kombinationen von mindestens zwei der vorgenannten weiteren Set-Komponenten ausgewählt ist.

## Revendications

1. Kit médical, en particulier, pour réaliser une thérapie intracorporelle, de préférence endoluminale et/ou endocavitaire, sous vide, comprenant :
- un dispositif de drainage de fluide (50) qui est adapté pour drainer des fluides corporels et étant entouré, au moins partiellement ou par sections, par une gaine (200),
- un dispositif médical (100) comprenant :
un premier tube flexible (10) ayant une partie d'extrémité proximale (12) et une partie d'extrémité distale (18), et
un deuxième tube flexible (20) ayant une partie d'extrémité proximale (22) et une partie d'extrémité distale (28),
dans lequel le premier tube flexible (10) est adapté pour insérer un endoscope (30) dans le corps d'un patient et le deuxième tube flexible (20) est adapté pour insérer le dispositif de drainage de fluide (50) dans le corps d'un patient, et dans lequel le premier tube flexible (10) et le deuxième tube flexible (20) sont raccordés entre eux dans le sens de la longueur.

2. Kit médical selon la revendication 1, dans lequel le dispositif médical (100) est **caractérisé en ce que** le deuxième tube flexible (20), en particulier la partie d'extrémité distale (28) du deuxième tube flexible (20), surplombe la partie d'extrémité distale (18) du premier tube flexible (10).

3. Kit médical selon la revendication 1 ou 2, **caractérisé en ce que** le dispositif médical (100) comprend en outre un premier élément de raccordement (14) et un deuxième élément de raccordement (16), dans lequel le premier élément de raccordement (14) est adapté pour raccorder, en particulier raccorder positivement et/ou raccorder par friction, une partie d'extrémité proximale de l'endoscope (30) à la partie d'extrémité proximale (12) du premier tube flexible (10) et le deuxième élément de raccordement (16) est adapté pour raccorder, en particulier lier matériellement, une partie d'extrémité distale (38) de l'endoscope (30) à la partie d'extrémité distale (28) du deuxième tube flexible (20) ou à une partie du deuxième tube flexible (20) positionnée de manière proximale par rapport à la partie d'extrémité distale (28) du deuxième tube flexible (20).

4. Kit médical selon l'une quelconque des revendications précédentes, **caractérisé en ce que** le dispositif médical (100) comprend en outre un élément de manipulation (40) adapté pour rétracter ou étirer la partie d'extrémité distale (28) du deuxième tube flexible (20).

5. Kit médical selon la revendication 4, dans lequel le dispositif médical (100) est **caractérisé en ce que** l'élément de manipulation (40) est sous la forme d'un élément longitudinal (214), en particulier un fil ou un fil métallique, fixé, en particulier cousu ou noué, à la partie d'extrémité distale (28) du deuxième tube flexible (20).

6. Kit médical selon la revendication 4 ou 5, dans lequel le dispositif médical (100) est **caractérisé en ce qu'**une partie d'extrémité distale (48) de l'élément de manipulation (40) a au moins une marque.

7. Kit médical selon l'une quelconque des revendications 4 à 6, **caractérisé en ce que** le dispositif médical (100) comprend en outre un troisième élément de raccordement (26) adapté pour raccorder une partie d'extrémité distale (48) de l'élément de manipulation (40) à la partie d'extrémité distale (28) du deuxième tube flexible (20) et pour déconnecter la partie d'extrémité distale (48) de l'élément de manipulation (40) de la partie d'extrémité distale (28) du deuxième tube flexible (20).

8. Kit médical selon l'une quelconque des revendications précédentes, **caractérisé en ce que** le dispositif médical (100) comprend en outre un élément de traction (80), en particulier sous la forme d'un fil métallique, adapté pour plier la partie d'extrémité distale (28) du deuxième tube flexible (20) dans la direction de la partie d'extrémité proximale (22) du deuxième tube flexible (20).

9. Kit médical selon l'une quelconque des revendications précédentes, **caractérisé en ce que** le dispositif médical (100) a en outre un élément de guidage d'insertion (60) situé à la partie d'extrémité proximale (22) du deuxième tube flexible (20) et adapté pour pousser le dispositif de drainage de fluide (50), en particulier le dispositif de drainage de fluide (50) étant entouré, au moins partiellement ou par sections, par une gaine (200), dans le deuxième tube flexible (20).

10. Kit médical selon l'une quelconque des revendications précédentes, **caractérisé en ce qu'**une poignée (90) du dispositif médical (100) ou de l'élément de guidage d'insertion (60) est adaptée pour être raccordée ou est raccordée au dispositif de drainage de fluide (50), en particulier à une partie d'extrémité proximale (52) du dispositif de drainage de fluide (50).

11. Kit médical selon l'une quelconque des revendications 3 à 10, dans lequel le dispositif médical (100) est **caractérisé en ce que** le premier élément de raccordement (14) et l'élément de guidage d'insertion (60) sont formés d'un seul tenant, dans lequel de préférence une partie d'extrémité distale du premier élément de raccordement (14) est insérée dans/à l'intérieur de la partie d'extrémité proximale (12) du premier tube flexible (10) et une partie d'extrémité distale de l'élément de guidage d'insertion (60) est insérée dans/à l'intérieur de la partie d'extrémité proximale (22) du deuxième tube flexible (20).

12. Kit médical selon l'une quelconque des revendications précédentes, **caractérisé en ce que** le dispositif de drainage de fluide (50) comprend un élément de collecte de fluide (53), en particulier ayant une structure mousseuse ou spongieuse, et un élément de communication de fluide (55).

13. Kit médical selon l'une quelconque des revendications précédentes, **caractérisé en ce que** la gaine (200) a un élément de libération (210) adapté pour libérer le dispositif de drainage de fluide (50) ou l'élément de collecte de fluide (53) de la gaine (200).

14. Kit médical selon l'une quelconque des revendications précédentes, **caractérisé en ce qu'**une queue longitudinale (220) est formée à une partie d'extrémité proximale (202) de la gaine (200) et/ou la gaine (200) a un marquage indiquant un début proximal de l'élément de libération (210).

15. Kit médical selon l'une quelconque des revendications précédentes, **caractérisé en ce que** le kit médical comprend au moins un composant de kit supplémentaire sélectionné dans le groupe comprenant un élément d'alimentation, un élément de manipulation, un élément de traction, au moins un élément de poussée, une pince, un endoscope, une seringue, un tube dilatateur extenseur, des connecteurs de tube tels qu'un connecteur Luer lock et/ou un connecteur en Y, un connecteur étant adapté pour être agencé entre l'élément de communication de fluide et un/le tube dilatateur extenseur, un lubrifiant, des éléments d'essuyage, des ciseaux, des instructions d'utilisation et des combinaisons d'au moins deux des composants de kit supplémentaire susmentionnés.
